# EUROPÄISCHE PATENTSCHRIFT

(11) **EP 1 276 720 B1**
(45) Veröffentlichungstag und Bekanntmachung des Hinweises auf die Patenterteilung: **20.12.2006**
(21) Anmeldenummer: 01947247.1
(22) Anmeldetag: 27.04.2001
(51) Int. Cl.: C07D 209/12

(54) **2-ACYL-INDOLDERIVATE UND DEREN VERWENDUNG ALS ANTITUMORMITTEL**
2-ACYL INDOL DERIVATIVES AND THEIR USE AS ANTI-TUMOUR AGENTS
DERIVES DE 2-ACYL-INDOLE ET LEUR UTILISATION COMME AGENTS ANTITUMORAUX

(30) Priorität: 28.04.2000 DE 10020852; 20.01.2001 DE 10102629
(43) Veröffentlichungstag der Anmeldung: 22.01.2003
(73) Patentinhaber: Baxter Healthcare S.A., 8304 Wallisellen (CH)
(72) Erfinder: BECKERS, Thomas, 60596 Frankfurt am Main (DE); BAASNER, Silke, 61137 Schöneck (DE); KLENNER, Thomas, 55218 Ingelheim (DE); MAHBOOBI, Siavosh, 93051 Regensburg (DE); PONGRATZ, Herwig, 93053 Regensburg (DE); FRIESER, Markus, 93142 Maxhütte-Rappenbügl (DE); HUFSKY, Harald, 85080 Gaimersheim (DE); HOCKEMEYER, Jörg, 28279 Bremen (DE); FIEBIG, Heinz-Herbert, 79110 Freiburg (DE); BURGER, Angelika, 79104 Freiburg (DE); BÖHMER, Frank-D., 07778 Dorndorf (DE)
(74) Vertreter: Hock, Joachim
(86) Internationale Anmeldenummer: PCT/EP2001/004783
(87) Internationale Veröffentlichungsnummer: WO 2001/082909

(56) Entgegenhaltungen:
- WO-A-98/11101
- WO-A-99/05104
- GB-A- 1 472 342
- GB-A- 2 283 745
- US-A- 3 660 430
- US-A- 3 838 167
- D. ST. BLACK ET. AL. : "A Simple Synthesis of 2-acyl Indoles from Isatins" JOURNAL OF THE CHEMICAL SOCIETY, CHEMICAL COMMUNICATIONS, Bd. 1980, 1980, Seite 200 XP001028985
- R. S. MALI ET. AL.: "Synthesis of 2-Acyl and 2-Benzoyl Indoles" JOURNAL OF CHEMICAL RESEARCH, SYNOPSIS, Bd. 2000, 2000, Seiten 8-9, XP001029293
- D. ST. BLACK ET. AL.: "Synthesis of a New Class of Indole-Containing Macrocycles" JOURNAL OF THE CHEMICAL SOCIETY, CHEMICLA COMMUNICATIONS, Bd. 1989, 1989, Seiten 425-6, XP001029425
- T. KAMETANI ET. AL.: "Nitrene. IX. The Reaction of beta-(2-Nitrobenzoyl)-2-nitrostyrene Derivatives with Triethyl Phosphite (Studies on the Syntheses of Heterocyclic Compounds). " YAKAGUKU ZASSHI, Bd. 91, Nr. 9, September 1971 (1971-09), Seiten 1033-6, XP001021190
- S. KAR ET. AL.: "Photochemical Intramolecular Cyclisation Routes to Heterocycles." JOURNAL OF THE INDIAN CHEMICAL SOCIETY, Bd. 76, Nr. 11, November 1999 (1999-11), Seiten 607-10, XP001028947
- R. AUMANN ET. AL.: "3-Amino-2-aroylindole, 2-Alkylideneindolenine, Pyrazino-diindole und Azetidine aus Arylisocyaniden und Carbenkomplexen" CHEMISCHE BERICHTE, Bd. 119, 1986, Seiten 2289-307, XP001029207
- G. W. GRIBBLE ET. AL.: "A Versatile and Efficient Construction of the 6H-Pyrido[4,3-b]carbazole Ring System. " JOURNAL OF ORGANIC CHEMISTRY, Bd. 57, Nr. 22, 23. Oktober 1992 (1992-10-23), Seiten 5891-9, XP001028945
- D. ST. BLACK ET. AL.: "Acid Catalysed Reaction of Activated Indoles with Methyl Ketones." TETRAHEDRON LETTERS, Bd. 32, Nr. 12, 1991, Seiten 1587-90, XP001021193
- C. D. JONES ET. AL.: "A Direct Synthesis of 2-Acylindoles." JOURNAL OF ORGANIC CHEMISTRY, Bd. 37, Nr. 23, 1972, Seiten 3622-3, XP002087982
- J. F. P. ANDREWS ET. AL.: "Pyrrole-2,3-quinodimethane Analogues in the Synthesis of Indoles. Part 2. Synthesis and Diels-Alder Reactions of 1,6-Dihydropyrano[4,3-b]pyrrol-6(IH)ones" TETRAHEDRON, Bd. 49, Nr. 33, 1993, Seiten 7353-72, XP001019138

## Beschreibung

Die Erfindung betrifft die Verwendung von Indol- und Heteroindolderivate der allgemeinen Formel 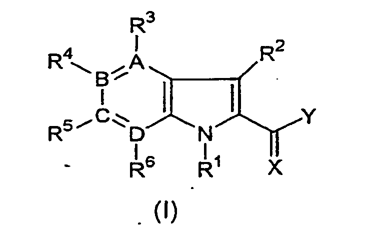 deren Tautomere, deren Stereoisomere, deren Gemische und deren Salze zur Behandlung von Tumorerkrunkungen bei Säugetieren, vorzugsweise beim Menschen.

Die Aufgabe der vorliegenden Erfindung liegt in der Bereitstellung von neuen Wirkstoffen für die Tumorbehandlung in Säugetieren.

In der deutschen Offenlegungschrift Nr. DE 2 501 468 werden 1-Alkyl-2-pyridinylcarbonyl-substituierte Indolverbindungen, deren Herstellung und deren Verwendung als Fibrinolytika oder Thrombolytika beschrieben. Eine Antitumorwirkung ist weder beschrieben noch nahegelegt.

In der belgischen Patentschrift Nr. BE 637355 werden 2-Benzoyl-substituierte Indolverbindungen als Zwischenprodukte in einer Grignard-Reaktion zu den entsprechenden 1-Amino-alkyl-1-Hydroxy-Derivaten (Phenylindolyl-alkanolamine) umgesetzt. Eine biologische Wirkung der Zwischenprodukte ist nicht beschrieben noch dem Durchschnittsfachmann nahegelegt.

In der deutschen Offenlegungsschrift Nr. DE 2 037 998 wird ein Verfahren zur Herstellung von 2-Benzoyl-, 2-Acetyl, 2-Propionyl und 2-p-Toluoylindol beschrieben, wobei die Klasse der 2-Acylindole als "verhältnismäßig unzugänglich" beschrieben wird. Auf die Verwendung der 2-Acylindole als Zwischenprodukte bei der Herstellung von Phenylindolyl-alkanolamine-Sedativa gemäß der vorstehend genannten belgischen Patentschrift Nr. 637 355 wird verwiesen. Die Verwendung der 2-Acylindole zur Herstellung von Farbstoffen, Alkaloiden, Pflanzenhormonen und Proteinen ist ohne nähere Angaben bloß erwähnt. Eine Verwendung der 2-Acylindole als Arzneimittel ist weder offenbart noch nahegelegt.

In der Publikation mit dem Titel "Nucleophilic Substitution of C-Hydrogen on the Five-membered Ring of Indoles" von John A. Joule in *Progress in Heterocyclic Chemistry,* 86VK, 7200.6-11, Seiten 45-65 wird auf Seite 50 die Herstellung von Hydroxy-2-indolyl-(2-Hydroxymethyl)phenyl-methan, auf Seite 54 die herstellung von 2-Benzoylindol und auf Seite 55 die Herstellung von 2-Cyclopropycarbonylindol beschrieben. Eine medizinosche Verwendung der genannten Verbindungen ist weder offenbart noch nahegelegt.

In der Publikation von David St. C. Black et al., *J. Chem. Soc., Chem. Commun.*, 1989, S. 425-426 wird die Herstellung von 2-(p-Chlorphenylcarbonyl-)-3-methyl-4,6-dimethoxy-indol und dessen Verwendung als Zwischenprodukt bei der Synthese Indolhaltiger Makrocyclen beschrieben.

In der am 02. Mai 19972 erteilten US Patentschrift Nr. 3,660,430 von Meier E. Freed et al. sind 3-Phenyl-substituierte 2-Benzoylindol-Verbindungen, deren Herstellung und deren Verwendung als ZNS Beruhigungsmittel beschrieben.

In der am 24. September 1974 erteilten US Patentschrift Nr. 3,838,167 von Charles D. Jones ist ein Verfahren zur Herstellung von 2-Acyl-indolverbindungen beschrieben. Als einziges Beispiel für ein in 3-Position unsubstituiertes 2-Benzoylindol wird 2-(3-Bromobenzoyl)-7-trifluormethylindol genannt. Bezüglich der Verwendung als ZNS Beruhigungsmittel wird auf die vorstehend genannte US Patentschrift 3,660,430 verwiesen.

In der Publikation von Michael D. Varney et al., J. Med. Chem. 1994, 37, Seiten 2274-2284 werden 2-Benzoyl- (meta-Position: H, Trifluormethyl oder Methyl) und 2-Cyclohexylcarbonyl-indolverbindungen als Zwischenprodukte zur Herstellung von HIV-Proteaseinhibitoren beschrieben. Eine biologische Wirkung für die Zwischenprodukte ist weder offenbart noch nahegelegt.

In der Publikation von Gordon W. Gribble et al., *J. Org. Chem. 1992, 57, 5891-*5899 werden 2-(2-Carboxy)-benzoyl- und in 5-Position mit Wasserstoff oder Methoxy substituierte 2-(5-Carboxy-pyridin-4-yl-indolderivate als Zwischenprodukte für die Synthese von Benzo[b]carbazol bzw. 6H-Pyrido[4,3-b]carbazolen beschrieben. Eine biologische Wirkung für die Zwischenverbindungen ist weder offenbart noch nahegelegt.

In der Publikation von S. Cenini, *Journal of Molecular Catalysis A: Chemical* 111 (1996) 37-41, ist die Palladium oder Ruthenium katalysierte Synthese von im Indolring unsubstituierten 2-Benzoylindolen beschrieben, wobei der Phenylring in den Positionen 3, 4 oder 5 mit Wasserstoff, Halogen, Methyl oder Methoxy substituiert ist. Eine biologische Wirkung für die hergestellten 2-Acylindole ist nicht offenbart.

In der Publikation von David St. C. Black und L. C.H. Wong, *J.C.S. Comm.* 1980, Seite 200 ist die Synthese von 2-Acylindolen beschrieben, welche in den Indolpositionen 4 bis 7 mit Chlor, Methyl oder Methoxy substituiert sind. Eine biologische Wirkung ist für die hergestellten 2-Acylindole ist weder offenbart noch nahegelegt.

In der Publikation von David St. C. Black et al., *Tetrahedron Letters,* Vol. 32, No. 12, Seiten 1587-1590, 1991 ist die Umsetzungsreaktion von 3-Methyl-4,7-dimethoxy-2-benzoylindol mit Methyliodid unter Bildung der entsprechenden Carbinolverbindung beschrieben. Eine biologische Wirkung für die Ausgangsverbindung ist weder offenbart noch nahegelegt.

In der Publikation von Tetsuji Kametani et al., Yakugaku-zasshi, 91 (9) 1033-1036 (1971) ist ein Verfahren zur Herstellung der Verbindung 2-Benzoyl-5,6-methylendioxy-indol aus β-(Benzoyl)-4,5-methylendioxy-2-nitro-styrol beschrieben.

In der Publikation von Charles D. Jones and Tulio Suarez, J. Org. Chem., Vol. 37, No. 23, 1972, Seiten 3622-3623 wird ein Verfahren zur Herstellung von 2-Acylindolen beschrieben. Eine biologische Wirkung ist für die hergestellten Verbindungen weder offenbart noch nahegelegt.

In der Publikation von V.I. Gorgos et al., Khimiya Geterotsiklicheskikh Soedinenii, No. 11, pp. 1490-1492.(englische Übersetzung in UDC 547.756'757.07; S. 1179-1182) wird ein Verfahren zur Herstellung von in 5- oder 7-Position mit Brom oder Methoxy substituierten 2-Benzoylindolen beschrieben. Eine biologische Wirkung ist für die hergestellten Verbindungen nicht offenbart. Gleiches gilt für den sowjetischen Erfinderschein Nr. 696016, in dem die Autoren der vorstehend genannten Publikation als Erfinder benannt sind.

In der Publikation von R. S. Mali et al., J. Chem. Research (S), 2000, 8-9 werden im Indolring substituierte 2-Benzoylindolverbindungen als Zwischenprodukte für die Synthese von Carbazolen und Pyridocarbazolen beschrieben. Eine biologische Wirkung ist für die hergestellten Verbindungen weder offenbart noch nahegelegt.

In der Publikation von S. Kar und S. Lahiri, J. Indian Chem. Soc., Vol. 76, Nov.-Dec. 1999, pp. 607-610 wird die Synthese von 2-Acroylindolen aus entsprechenden 2-Azidobenzaldehyden beschrieben. Eine biologische Wirkung ist der so hergestellten Verbindungen ist weder offenbart noch nahegelegt.

In der Publikation von R. Aumann und H. Heinen, Chem. Ber. 119, 2289-2307 (1986) wird die Synthese von 3-Amino-2-aroylindolen beschrieben. Eine biologische Wirkung ist für die hergestellten Verbindungen weder offenbart noch nahegelegt.

In der Publikation von C.D. Jones und T. Suärez, J. Org. Chem., Vorl. 37, No. 23, 3622-3623 (1972) wird eine Synthese von 2-Acylindolen als Ausgangsverbindungen für weitere Synthesen beschrieben. Eine biologische Wirkung ist für die hergestellten 2-Acylindole weder offenbart noch nahegelegt.

In der Publikation von J.F.P. Andrews et al., Tetrahedron Vol. 49, No. 33, pp. 7353-7372 (1993) wird die Diels-Alder-Reaktion von Pyrano[4,3-b]pyrrol-6-onen mit Alkenen und Alkinen zu 2-Acylindolen beschrieben. Eine biologische Wirkung ist für die hergestellten Verbindungen weder offenbart noch nahegelegt.

In der britischen Patentschrift GB 1 472 342 werden 2-(2-pyridylcarbonyl)-indole und deren Herstellung beschrieben. Eine Antitumor-Wirkung ist für die hergestellten Verbindungen weder offenbart noch nahegelegt.

In der Internationalen Patentanmeldung WO 98/11101 (PCT/NZ/00117) sind Cyclopropylindol-Verbindungen beschrieben, die zwar eine 2-Acylindol-Untereinheit enthalten, aber ansonsten strukturell keine Ähnlichkeit mit den 2-Acyl-Indolderivaten der vorliegenden Erfindung aufweisen.

Überraschenderweise wurde nun gefunden, daß die Verbindungen der allgemeinen Formel I 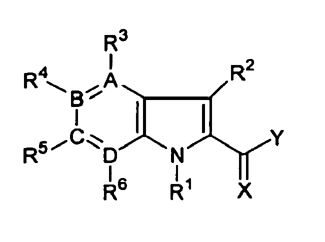 worin
- R1: Wasserstoff, (C1-C6)-Alkylcarbonyl, vorzugsweise Acetyl, (C₁-C₆)-Alkyl, Mono-(C₁-C₆)-Alkylamino-(C₁-C₄)-alkyl, Di-(C₁-C₆)-Alkylamino-(C₁-C₄)-alkyl, wobei die beiden (C₁-C₆)-Alkylreste miteinander einen Ring bilden können, welcher gegenbenenfalls ein oder mehrere NH, N-(C1-C6)-Alkyl, O oder S-Glieder aufweist, (C6-C14)-Aryl-(C1-C6)-alkyl oder (C6-C14)-Aryl-(C1-C6)-alkoxy-(C1-C6)-alkyl bedeutet;
- R2: ein Wasserstoffatom, Halogen, Cyano, Nitro, (C1-C6)-Alkyl, mit ein oder mehreren Halogenatomen substituiertes (C1-C6)-Alkyl, vorzugsweise Trifluormethyl, mit ein oder mehreren Halogenatomen substituiertes (C1-C6)-Alkoxy, vorzugsweise Trifluormethoxy, (C2-C6)-Alkenyl, (C2-C6)-Alkinyl, (C3-C8)-Cycloalkyl, (C1-C6)-Alkoxy, (C1-C6)-Alkoxycarbonyloxy, (C1-C6)-Alkylcarbonyloxy, (C1-C4)-Alkylthio, (C1-C4)-Alkylsulfinyl, (C1-C4)-Alkylsulfonyl, (C1-C6)-Alkoxy-(C1-C6)-alkyl, Amino, Mono-(C1-C6)-Alkylamino, Di-N,N-(C1-C6)-alkyl-amino, wobei die beiden (C1-C6)-Alkylreste miteinander einen Ring bilden können, welcher gegenbenenfalls ein oder mehrere NH, N-(C1-C6)-Alkyl, O oder S aufweist, (C6-C14)-Aryl, (C6-C14)-Aryloxy, (C6-C14)-Aryl-(C1-C4)-alkyl, (C6-C14)-Aryl-(C1-C4)-alkoxy-(C1-C4)-alkyl, (C1-C6)-alkylcarbonyl, (C1-C6)-Alkoxycarbonyl, oder Hydroxyl bedeutet;
- A, B, C, D: unabhängig voneinander für ein Stickstoffatom (wobei R3, R4, R5 und R6 dann für das freie Elektronenpaar am Stickstoffatom stehen) oder ein mit einem der Reste R3-R6 substituiertes Kohlenstoffatom stehen;
- R3, R4, R5 und R6: unabhängig voneinander, wenn an Stickstoff gebunden, ein freies Elektronenpaar, oder, wenn an Kohlenstoff gebunden, Wasserstoff, Halogen, Cyano, Nitro, geradkettig oder verzweigtes (C1-C6) Alkyl, mit einem oder mehreren Halogenatomen substituiertes geradkettig oder verzweigtes (C1-C6)-Alkyl, vorzugsweise Trifluormethyl, mit einem oder mehreren Halogenatomen substituiertes geradkettig oder verzweigtes (C1-C6)-Alkoxy, vorzugsweweise Trifluormethoxy, (C2-C6)-Alkenyl, (C2-C6)-Alkinyl, (C3-C8)-Cycloalkyl, geradkettig oder verzweigtes (C1-C6)-Alkoxy, vorzugsweise Methoxy, geradkettig oder verzweigtes (C1-C6)-Alkylendioxy, vorzugsweise Methylendioxy, (C1-C6)-Alkoxycarbonyloxy, (C1-C6)-Alkylcarbonyloxy, (C1-C4)-Alkylthio, (C1-C4)-Alkylsulfinyl, (C1-C4)-Alkylsulfonyl, Carboxy, Carboxy-(C1-C6)-alkylester, Carboxamid, N-(C1-C4)-Alkyl-carboxamid, N,N-di-(C1-C4)-alkyl-carboxamid, (C1-C6)-Alkoxy-(C1-C6)-alkyl, Amino, Mono-(C1-C6)-Alkylamino, N,N-di-(C1-C6)-alkyl-amino wobei die beiden C1-C6-Alkylreste miteinander einen Ring bilden können, welcher gegenbenenfalls ein oder mehrere NH, N-(C1-C6)-Alkyl, O oder S aufweist, (C6-C14)-Aryl, (C6-C14)-Aryloxy, (C6-C14)-Aryl-(C1-C4)-alkyl, (C6-C14)-Aryl-(C1-C4)-alkoxy-(C1-C4)-alkyl, (C1-C6)-Alkylcarbonyl, (C1-C6)-Alkylcarbonyloxy, (C1-C6)-Alkoxycarbonyl, Hydroxyl, wobei zwei direkt benachbarte Reste miteinander verbunden sein können, bedeuten;
- Y: unsubstituiertes oder ganz oder teilweise gleich oder verschieden substituiertes (C6-C14)-Aryl, vorzugsweise Phenyl oder 1- oder 2-Naphthyl, oder unsubstituiertes oder ganz oder teilweise gleich oder verschieden mindestens ein bis vier N, NH, N-(C1-C6)-alkyl, O und/oder S als Ringglieder aufweisendes (C1-C13)-Heteroaryl oder unsubstituiertes oder ganz oder teilweise gleich oder verschieden substituiertes (C3-C8)-Cycloalkyl, wobei die gleichen oder verschiedenen Substituenten unabhängig voneinander ausgewählt sind aus der Gruppe bestehend aus Halogen, vorzugsweise Fluor, Chlor, Brom oder Jod; Cyano; geradkettig oder verzweigtes Cyano-(C1-C6)-alkyl; Hydroxy; mit ein oder mehreren Hydroxy substituiertes geradkettig oder verzweigtes (C1-C6)-Alkyl; Carboxy; Carboxy-(C1-C6)-alkylester, Carboxamid; N-(C1-C6)-Alkyl-carboxamid, N,N-di-(C1-C4)-alkyl-carboxamid, Nitro, geradkettig oder verzweigtes (C1-C6)-Alkyl, mit einem oder mehreren Halogenatomen substituiertes geradkettig oder verzweigtes (C1-C6)-Alkyl, vorzugsweise Trifluormethyl, mit einem oder mehreren Halogenatomen substituiertes geradkettig oder verzweigtes (C1-C6)-Alkoxy, vorzugsweise Trifluormethoxy, geradkettig oder verzweigtes (C2-C6)-Alkenyl, geradkettig oder verzweigtes (C2-C6)-Alkinyl, (C3-C8)-Cycloalkyl, geradkettig oder verzweigtes (C1-C6)-Alkoxy, vorzugsweise Methoxy, geradkettig oder verzweigtes (C1-C6)-Alkylendioxy, vorzugsweise Methylendioxy, Thio (-SH), geradkettig oder verzweigtes (C1-C6)-Alkylthio, (C1-C6)-Alkylsulfinyl, (C1-C6)-Alkylsulfonyl, (C1-C6)-Alkoxy-(C1-C6)-alkyl, Amino, geradkettig oder verzweigtes Mono-(C1-C6)-Alkylamino, geradkettig oder verzweigtes N,N-di-(C1-C6)-alkyl-amino wobei die beiden (C1-C6)-Alkylreste miteinander einen Ring bilden können, welcher gegenbenenfalls ein oder mehrere NH, N-(C1-C6)-Alkyl, O und/oder S aufweisen kann, (C6-C14)-Aryl, (C6-C14)-Aryloxy, (C6-C14)-Aryl-(C1-C6)-alkyl, (C6-C14)-Aryl-(C1-C6)-alkoxy-(C1-C6)-alkyl, (C1-C6)-Alkylcarbonyl, (C1-C6)-Alkylcarbonyloxy, (C1-C6)-Alkoxycarbonyl, (C1-C6)-Alkoxycarbonyloxy, geradkettig oder verzweigtes Mono- und N,N-di-(C1-C6)-Alkylcarbonyl-amino, geradkettig oder verzweigtes Mono- und N,N-di-(C1-C6)-Alkoxycarbonylamino, geradkettig oder verzweigtes N-(C1-C6)-alkylcarbonyl-N-(C1-C6)-alkylamino, geradkettig oder verzweigtes N-(C1-C6)-alkoxycarbonyl-N-(C1-C6)-alkyl-amino, Formylamino, Formyl, wobei zwei direkt benachbarte Reste miteinander verbunden sein können, bedeutet;
- X: für ein Sauerstoff- oder Schwefelatom, für NH, oder für eine geminal (am gleichen C-Atom) substituiertes Hydroxy und Wasserstoff (-CH(OH)-)steht;
deren Stereoisomere, deren Tautomere, deren Gemische sowie die pharmazeutisch verträglichen Salze davon, zur Herstellung eines Arzneimittels zur Behandlung von Tumorerkrankungen in Säugetieren verwendet werden können.

Gemäß einer besonderen Ausführungsform der vorliegenden Erfindung wird die Verwendung von mindestens einer Verbindung der allgemeinen Formel I dadurch charakterisiert, daß R1-R6, A, B, C, D, X und Y die vorstehend genannte Bedeutung haben, mit der Maßgabe, daß mindestens einer der Reste R3-R6 für geradkettig oder verzweigtes (C1-C6)-Alkoxy, vorzugsweise Methoxy; geradkettig oder verzweigtes (C1-C6)-Alkyl, vorzugsweise Methyl; geradkettig oder verzweigtes (C1-C6)-Alkylendioxy, vorzugsweise Methylendioxy, Hydroxyl; mit einem oder mehreren Halogenatomen substituiertes geradkettig oder verzweigtes (C1-C6)-Alkoxy, vorzugsweise Trifluormethoxy; mit einem oder mehreren Halogenatomen substituiertes geradkettig oder verzweigtes (C1-C6)-Alkyl, vorzugsweise Trifluormethyl; steht, bereitgestellt.

Gemäß einer weiteren Ausführungsform der Erfindung wird die Verwendung von mindestens einer Verbindung der allgemeinen Formel 1 dadurch gekennzeichnet, daß R1, R2, R3, R5, R6, A, B, C, D, X und Y die vorstehend genannte Bedeutung haben und der Rest R4 für geradkettig oder verzweigtes (C1-C6)-Alkoxy, vorzugsweise Methoxy; geradkettig oder verzweigtes (C1-C6)-Alkyl, vorzugsweise Methyl; geradkettig oder verzweigtes (C1-C6)-Alkylendioxy (wobei das zweite Sauerstoffatom wahlweise der Rest R4 oder R6 sein kann); vorzugsweise Methylendioxy, Hydroxyl; mit einem oder mehreren Halogenatomen substituiertes geradkettig oder verzweigtes (C1-C6)-Alkoxy, vorzugsweise Trifluormethoxy; mit einem oder mehreren Halogenatomen substituiertes geradkettig oder verzweigtes (C1-C6)-Alkyl, vorzugsweise Trifluormethyl; steht, bereitgestellt.

Gemäß einer weiteren Ausführungsform der Erfindung wird die Verwendung von mindestens einer Verbindung der obengenannten allgemeinen Formel 1, dadurch gekennzeichnet, daß R1, R2, R3, R5, R6, A, B, C, D, X und Y die vorstehend genannte Bedeutung haben und der Rest R4 für geradkettig oder verzweigtes (C1-C6)-Alkoxy, vorzugsweise Methoxy, steht, bereitgestellt.

Gemäß einer weiteren Ausführungsform der Erfindung wird die Verwendung von mindestens einer Verbindung der obengenannten allgemeinen Formel I nach einer der vorstehenden Ausführungsformen, dadurch gekennzeichnet, daß R1, R2, R3, R5, R6, A, B, C, D, X und Y die vorstehend genannte Bedeutung haben und der Rest R4 für Methoxy steht, bereitgestellt.

Gemäß einer weiteren Ausführungsform der Erfindung wird die Verwendung von mindestens einer Verbindung der allgemeinen Formel I nach einer der vorstehenden Ausführungsformen, dadurch gekennzeichnet, daß R1-R6, A, B, C, D und X die vorstehend genannte Bedeutung haben und der Rest Y für substituiertes oder unsubstituiertes (C6-C14)-Aryl oder mindestens ein bis vier N, NH, O und/oder S als Ringglied aufweisendes (C1-C13)-Heteroaryl steht, bereitgestellt.

Gemäß einer weiteren Ausführungsform der Erfindung wird die Verwendung von mindestens einer Verbindung der allgemeinen Formel I nach einer der vorstehenden Ausführungsformen, dadurch gekennzeichnet, daß R1-R6, A, B, C, D und X die vorstehend genannte Bedeutung haben und der Rest Y für (C6-C14)-Aryl oder mindestens ein N, NH, O und/oder S als Ringglied aufweisendes (C1-C13)-Heteroaryl, welches unsubstituiert oder mit mindestens einem Rest ausgewählt aus der Gruppe bestehend aus Wasserstoff, Amino, Halogen, Nitro, Cyano, geradkettig oder verzweigtes (C1-C6)-Alkoxy, vorzugsweise Methoxy; geradkettig oder verzweigtes (C1-C6)-Alkyl, vorzugsweise Methyl; Hydroxy; (C1-C6)-Alkylcarbonyloxy, (C1-C6)-Alkoxycarbonyloxy; mit einem oder mehreren Halogenatomen substituiertes geradkettig oder verzweigtes (C1-C6)-Alkoxy, vorzugsweise Trifluormethoxy; mit einem oder mehreren Halogenatomen substituiertes geradkettig oder verzweigtes (C1-C6)-Alkyl, vorzugsweise Trifluormethyl, substituiert ist, steht, bereitgestellt.

Gemäß einer weiteren Ausführungsform der Erfindung wird die Verwendung von mindestens einer Verbindung der allgemeinen Formel I nach einer der vorstehenden Ausführungsformen, dadurch gekennzeichnet, daß R1-R6, A, B, C, D und X die vorstehend genannte Bedeutung haben und der Rest Y für einen 1-Phenyl-Rest, welcher unsubstituiert oder mit Wasserstoff, 3,4-Dichlor, 2- oder 3-Methoxy, 2,4-Dimethoxy, 3-Nitro 3-Trifluormethyl, 2,3,4-Trimethoxy, 3,4,5-Trimethoxy substituiert ist, steht, bereitgestellt.

Gemäß einer weiteren Ausführungsform der Erfindung wird die Verwendung einer Verbindung der allgemeinen Formel I gemäß einer der vorstehenden Ausführungsformen zur Herstellung eines Medikaments mit antimitotischer Wirkung in Säugetieren bereitgestellt.

Gemäß einer weiteren Ausführungsform der Erfindung wird die Verwendung einer Verbindung der allgemeinen Formel I gemäß einer der vorstehenden Ausführungsformen zur Herstellung eines Medikaments zur direkten und/oder indirekten Inhibierung der Tubulinpolymerisation in Säugetierzellen, bereitgestellt.

Gemäß einer weiteren Ausführungsform der Erfindung wird die Verwendung einer Verbindung der allgemeinen Formel I gemäß einer der vorstehenden Ausführungsformen zur Herstellung eines Medikaments zur oralen, parenteralen oder topischen Behandlung von Tumorerkrankungen bei Säugetieren, vorzugsweise beim Menschen, bereitgestellt.

Im Rahmen der vorliegenden Erfindung wird ein Verfahren zur Herstellung der erfindungsgemäßen Verbindungen der Formel I, gekennzeichnet durch die Schritte
a) Lithiierung des entsprechenden 1-N-geschützten Indol- oder Heteroindolderivats und Umsetzung mit Z-CO-Y, wobei Z für eine geeignete Abgangsgruppe wie Halogen steht, oder H-CO-Y unter Erhalt des entsprechenden Methanonderivats bzw. des entsprechend tertiären Alkohols, welcher gegebenenfalls zum Methanonderivat oxidiert werden kann,
b) gegebenenfalls Abspaltung der Schutzgruppe und
c) gegebenenfalls weitere Umsetzung der reaktiven Reste nach an sich bekannten Verfahrensweisen, bereitgestellt.

Gemäß einem weiteren Aspekt der Erfindung werden die erfindungsgemäß verwendeten Antitumormittel, enthaltend eine wirksame Menge von mindestens einer Verbindung der Formel I, zusammen mit üblichen pharmazeutischen Hilfs- und/oder Trägerstoffen bereitgestellt.

Die Verbindungen gemäß Formel I lassen sich nach an sich bekannten Verfahren herstellen, beispielsweise nach folgenden Verfahren:
a) Lithiierung der Indolderivate und Umsetzung zu den entsprechenden Methanonen: 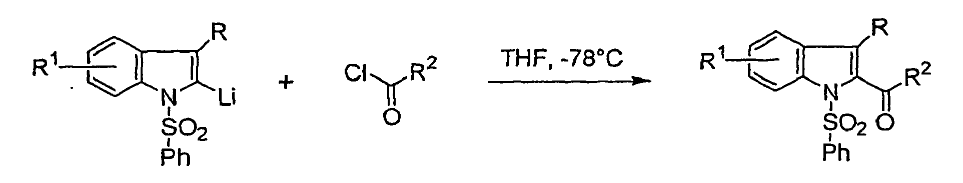 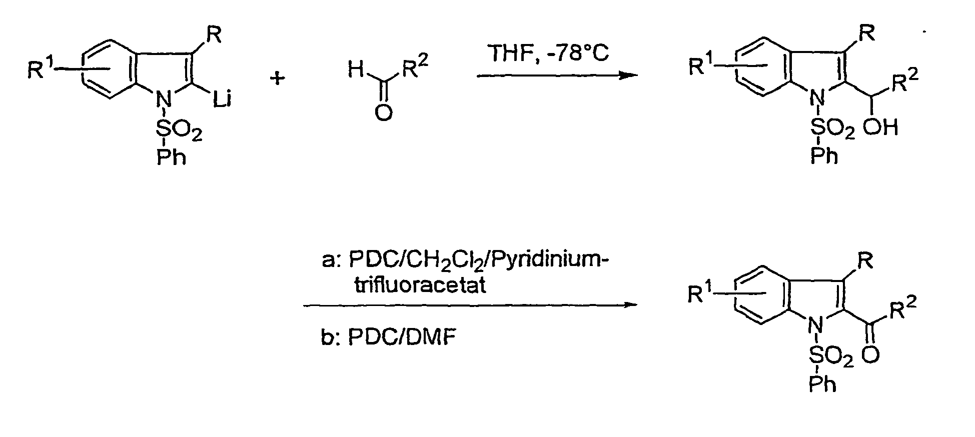
b) Entfernung der Phenylsulfonyl-Schutzgruppe: 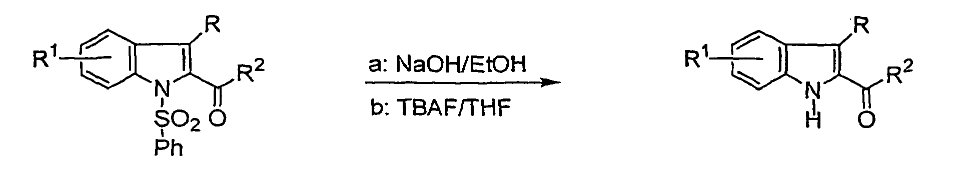
c) Weitere Umsetzung der Methanone für R¹ = 5-Benzyloxy:

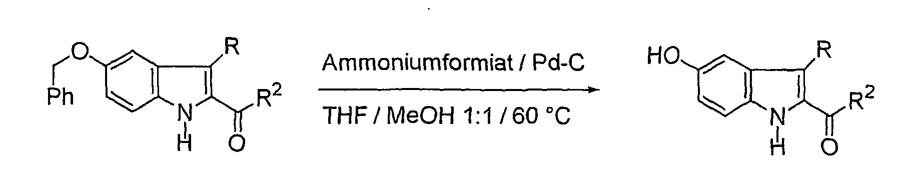

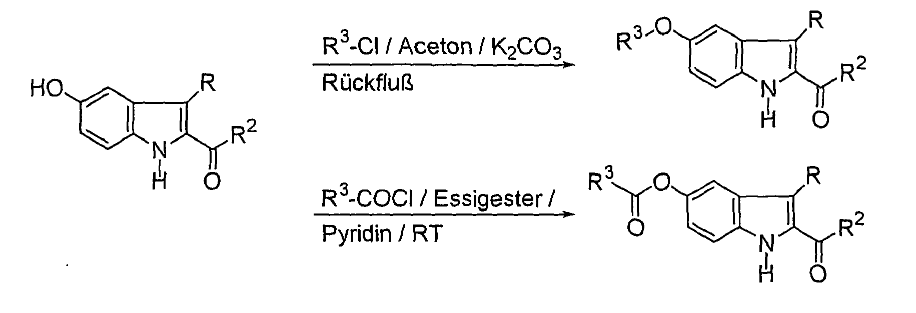

Die Verbindungen der obigen allgemeinen Formel I, in denen R1 für einen Wasserstoff oder einen Phenylsulfonyl-Rest steht, stellen wertvolle Zwischenprodukte zur Herstellung der übrigen Verbindungen der allgemeinen Formel I dar.

Die als Ausgangsstoffe verwendeten Verbindungen, welche teilweise im Handel erhältlich oder literaturbekannt sind, erhält man nach literaturbekannten Verfahren, des weiteren wird ihre Herstellung in den Beispielen beschrieben. Die literaturbekannten Verfahren sind beispielsweise in L. and M. Fieser, *Organische Chemie,* 2. Auflage, 1979, Seiten 1417 bis 1483 sowie in der dort auf den Seiten 1481-1483 zitierten Literatur, *Houben-Weyl-Müller,* Methoden der organischen Chemie und *Ullmanns Encyklopädie der technischen Chemie* beschrieben.

Ferner können die erhaltenen Verbindungen der allgemeinen Formel I in ihre Enantiomeren und/oder Diastereomeren aufgetrennt werden. So lassen sich beispielsweise die erhaltenen Verbindungen der allgemeinen Formel I, welche als Racemat auftreten, nach an sich bekannten Methoden in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren aufgetrennen, die, falls sie in racemischer Form anfallen, anschließend wie oben erwähnt in die Enantiomere getrennt werden können.

Die Enantiomerentrennung erfolgt vorzugsweise durch Säulentrennung an chiralen Phasen oder durch Umkristallisation aus einem optisch aktiven Lösungsmittel oder durch Umsetzen mit einer, mit der racemischen Verbindung Salze oder Derivate wie z.B. Ester oder Amide bildenden optisch aktiven Substanz.

Desweiteren können die erhaltenen Verbindungen der Formel I in ihre Salze, insbesondere für die pharmazeutische Anwendung in ihre pharmakologisch und physiologisch verträglichen Salze mit anorganischen oder organischen Säuren überführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die Verbindungen der Formel I, falls diese eine saure Gruppe wie eine Carboxylgruppe enthalten, gewünschtenfalls in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die Verbindungen der allgemeinen Formel I und deren Salze wertvolle Eigenschaften auf. So besitzen die erfindungsgemäß verwendeten Verbindungen der Formel I wertvolle pharmakologische Eigenschaften als Antitumormittel und bei der Chemotherapie von Tumorpatienten einsetzbar. Die Verbindungen der Formel I inhibieren die Zellteilung (Anti-Mitose-Wirkung) und hierdurch das Tumorwachstum. Die erfindungsgemäß verwendeten Verbindungen können darüberhinaus indirekt oder direkt die Tubulinpolymerisiation inhibieren. Die Inhibition der Zellteilung kann über eine Arretierung der Tumorzellen im Zellzyklus erfolgen, der dann zu einem Absterben der Zellen (Apoptose) führt. Weiterhin sind die Verbindungen der Formel 1 zur Verhinderung bzw. Verringerung der Metastasenbildung und -verbreitung im Körper geeignet. Sie besitzen außerdem ein anti-angiogenes Potential und können so über Hemmung der Tumorvaskularisierung als Antitumorwirkstoffe anwendbar sein.

Die folgenden Beispiele erläutern die Erfindung ohne sie zu beschränken.

### Allgemeine Vorschrift zur Herstellung der erfindungsgemäß verwendeten 1-Phenylsulfonyl-1H-2-indolylphenyl-1-methanole

Bei -78 °C werden zu 2.23 ml (15.9 mmol) abs. Diisopropylamin in 15 ml abs. THF 9.9 ml (15.9 mmol) n-Butyllithium getropft. Nach 10 min rühren bei dieser Temperatur wird auf 0 °C erwärmt und 30 min weitergerührt. Eine Lösung des entsprechenden 1-Phenylsulfonylindols (Komponente A) (14.0 mmol) in 22 ml abs. THF wird innerhalb 10 min zugegeben. Das Reaktionsgemisch wird 30 min bei 0 °C gerührt und anschließend auf -78 °C abgekühlt. Der entsprechende Aldehyd (Komponente B) (15.4 mmol) wird in 15 ml abs THF gelöst und tropfenweise zugegeben. Nach Erwärmen auf Raumtemperatur (über Nacht) wird das Gemisch auf auf 100 ml 1 % HCl gegossen. Die organische Phase wird abgetrennt, die Wasserphase drei mal mit je 50 ml Ethylacetrat extrahiert. Die vereinigten organischen Phasen werden mit 10 % Natriumhydrogencarbonatlösung und Wasser gewaschen und über Natriumsulfat getrocknet. Nach dem Abziehen des Lösungsmittels wird das Rohprodukt säulenchromatographisch gereinigt oder aus Ethanol umkristallisiert.

### Beispiel 1:

Komponente A: 5-Methoxy-1-phenylsulfonyl-1*H*-2-indol
Komponente B: Benzaldehyd
5-Methoxy-1-phenylsulfonyl-1*H*-2-indolylphenyl-1-methanol
Schmp.: 51-52 °C

### Beispiel 2:

Komponente A: 5-Methoxy-1-phenylsulfonyl-1*H*-2-indol
Komponente B: 2-methoxy-benzaldehyd
5-Methoxy-1-phenylsulfonyl-1*H*-2-indolyl(2-methoxyphenyl)-1-methanol
Schmp.: 75-76 °C

### Beispiel 3:

Komponente A: 5-Methoxy-1-phenylsulfonyl-1*H*-2-indol
Komponente B: 3-methoxy-benzaldehyd
5-Methoxy-1-phenylsulfonyl-1*H*-2-indolyl(3-methoxyphenyl)-1-methanol
Schmp.: 121-122 °C

### Beispiel 4:

Komponente A: 5-Methoxy-1-phenylsulfonyl-1*H*-2-indol
Komponente B: 4-methoxy-benzaldehyd
5-Methoxy-1-phenylsulfonyl-1*H*-2-indolyl(4-methoxyphenyl)-1-methanol
Schmp.: 78-79 °C

### Beispiel 5:

Komponente A: 5-Methoxy-1-phenylsulfonyl-1 H-2-indol
Komponente B: 2,4-dimethoxy-benzaldehyd
5-Methoxy-1-phenylsulfonyl-1*H*-2-indolyl(2,4-dimethoxyphenyl)-1-methanol
Schmp.: 119-120 °C

### Beispiel 6:

Komponente A: 1-Phenylsulfonyl-1*H*-2-indol
Komponente B: 3-pyridinyl-carbaldehyd
1-Phenylsulfonyl-1*H*-2-indolyl(3-pyridinyl)-1-methanol
Schmp.: 146 °C (Zers.).

### Beispiel 7:

Komponente A: 4-Hydroxy(1-phenylsulfonyl-1*H*-2-indol)
Komponente B: 4-cyanobenzaldehyd
4-Hydroxy(1-phenylsulfonyl-1*H*-2-indolyl)methyl-1-benzenecarbonitril
Schmp.: 150 °C (Zers.)

### Beispiel 8:

Komponente A: 5-methoxy-1-phenylsulfonyl-1 H-2-indol
Komponente B: 4-Isochinolinyl-carbaldehyd
4-Isochinolinyl(5-methoxy-1-phenylsulfonyl-1*H*-2-indolyl)-1-methanol
Schmp.: 138-139 °C

### Beispiel 9:

Komponente A: 5-methoxy-1-phenylsulfonyl-1*H*-2-indol
Komponente B: 1-Isochinolinylcarbaldehyd
1-Isochinolinyl(5-methoxy-1-phenylsulfonyl-1*H*-2-indolyl)-1-methanol
Schmp.: 167-168 °C

### Allgemeine Vorschrift zur Herstellung der erfindungsgemäß verwendeten 1-Phenylsulfonyl-1H-2-indolylphenyl-1-methanone

Zu 4.01 ml (28.6 mmol) abs. Diisopropylamin in 30 ml abs. THF werden 17.8 ml (28.6 mmol) n-Butyllithium getropft. Nach 10 min rühren bei dieser Temperatur wird auf 0 °C erwärmt. Eine Lösung des entsprechenden 1-Phenylsulfonylindols (Komponente A) (26.0 mmol) in 35 ml abs. THF wird innerhalb 10 min zugegeben.

Das Reaktionsgemisch wird 60 min bei 0 °C gerührt und anschließend auf -78 °C abgekühlt.
Dieses Gemisch wird zu einer auf -78 °C vorgekühlten Lösung des entsprechenden Carbonsäurechlorids (Komponente B) (30 mmol) in 40 ml abs. THF gegeben. Nach 60 min rühren bei dieser Temperatur wird der Ansatz auf 200 ml 5 % Natriumhydrogencarbonat-Lösung gegossen und mit Ethylacetat extrahiert. Die organische Phase wird über Natriumsulfat getrocknet und das Lösungsmittel abgezogen. Der Rückstand wird in Ether gelöst und bis zur beginnenden Kristallisation mit Petrolether versetzt. Das Produkt wird abfiltriert, mit Petrolether gewaschen und getrocknet.

### Beispiel 10:

Komponente A: 1-Phenylsulfonyl-1 H-2-indol
Komponente B: Benzoesäurechlorid
1-Phenylsulfonyl-1*H*-2-indolylphenyl-1-methanon
Schmp.: 142-143 °C

### Beispiel 11:

Komponente A: 1-Phenylsulfonyl-1*H*-2-indol
Komponente B: 2-methoxy-benzoesäurechlorid
1-Phenylsulfonyl-1*H*-2-indolyl(2-methoxyphenyl)-1-methanon
Schmp.: 141-143 °C

### Beispiel 12:

Komponente A: 1 -Phenylsulfonyl-1 H-2-indol
Komponente B: 3-methoxy-benzoesäurechlorid
1-Phenylsulfonyl-1*H*-2-indolyl(3-methoxyphenyl)-1-methanon
Schmp.: 101-103 °C

### Beispiel 13:

Komponente A: 1-Phenylsulfonyl-1*H*-2-indol
Komponente B: 2,4-dimethoxy-benzoesäurechlorid
1-Phenylsulfonyl-1*H*-2-indolyl(2,4-dimethoxyphenyl)-1-methanon
Schmp.: 66-68 °C

### Beispiel 14:

Komponente A: 1-Phenylsulfonyl-1*H*-2-indol
Komponente B: 3,4,5-trimethoxy-benzoesäurechlorid
1-Phenylsulfonyl-1*H*-2-indolyl(3,4,5-trimethoxyphenyl)-1-methanon
Schmp.: 152-153 °C

### Beispiel 15:

Komponente A: 3-Methyl-1-phenylsulfonyl-1*H*-2-indol
Komponente B: 2-methoxy-benzoesäurechlorid
3-Methyl-1-phenylsulfonyl-1*H*-2-indolyl(2-methoxyphenyl)-1-methanon
Schmp.: 167-169 °C

### Beispiel 16:

Komponente A: 3-Methyl-1-phenylsulfonyl-1*H*-2-indol
Komponente B: 3-methoxy-benzoesäurechlorid
3-Methyl-1-phenylsulfonyl-1*H*-2-indolyl(3-methoxyphenyl)-1-methanon
Schmp.: 113 °C

### Beispiel 17:

Komponente A: 3-Methyl-1-phenylsulfonyl-1*H*-2-indol
Komponente B: 2,4-dimethoxy-benzoesäurechlorid
3-Methyl-1-phenylsulfonyl-1*H*-2-indolyl(2,4-dimethoxyphenyl)-1-methanon
Schmp.:155-157 °C

### Beispiel 18:

Komponente A: 3-Methyl-1-phenylsulfonyl-1*H*-2-indol
Komponente B: 3,4,5-trimethoxy-benzoesäurechlorid
3-Methyl-1-phenylsulfonyl-1*H*-2-indolyl(3,4,5-trimethoxyphenyl)-1-methanon

### Beispiel 19:

Komponente A: 5-Methyl-1-phenylsulfonyl-1*H*-2-indol
Komponente B: 2-methoxy-benzoesäurechlorid
5-Methyl-1-phenylsulfonyl-1*H*-2-indolyl(2-methoxyphenyl)-1-methanon
Schmp.: 157-158 °C

### Beispiel 20:

Komponente A: 5-Methyl-1-phenylsulfonyl-1*H*-2-indol
Komponente B: 3-methoxy-benzoesäurechlorid
5-Methyl-1-phenylsulfonyl-1*H*-2-indolyl(3-methoxyphenyl)-1-methanon
Schmp.: 124-127 °C

### Beispiel 21:

Komponente A: 5-Methyl-1-phenylsulfonyl-1*H*-2-indol
Komponente B: 2,4-dimethoxy-benzoesäurechlorid
5-Methyl-1-phenylsulfonyl-1*H*-2-indolyl(2,4-dimethoxyphenyl)-1-methanon

### Beispiel 22:

Komponente A: 5-Methyl-1-phenylsulfonyl-1*H*-2-indol
Komponente B: 3,4,5-trimethoxy-benzoesäurechlorid
5-Methyl-1-phenylsulfonyl-1*H*-2-indolyl(3,4,5-trimethoxyphenyl)-1-methanon

### Beispiel 23:

Komponente A: 5-Methoxy-1-phenylsulfonyl-1*H*-2-indol
Komponente B: Benzoesäurechlorid
5-Methoxy-1-phenylsulfonyl-1*H*-2-indolylphenyl-1-methanon
Schmp.: 148°C

### Beispiel 24:

Komponente A: 5-Methoxy-1-phenylsulfonyl-1*H*-2-indol
Komponente B: 2-methoxy-benzoesäurechlorid
5-Methoxy-1-phenylsulfonyl-1*H*-2-indolyl(2-methoxyphenyl)-1-methanon
Schmp.: 179 °C

### Beispiel 25:

Komponente A: 5-Methoxy-1-phenylsulfonyl-1 H-2-indol
Komponente B: 3-methoxy-benzoesäurechlorid
5-Methoxy-1-phenylsulfonyl-1*H*-2-indolyl(3-methoxyphenyl)-1-methanon
Schmp.: 181 °C

### Beispiel 26:

Komponente A: 5-Methoxy-1-phenylsulfonyl-1 H-2-indol
Komponente B: 4-methoxy-benzoesäurechlorid
5-Methoxy-1-phenylsulfonyl-1*H*-2-indolyl(4-methoxyphenyl)-1-methanon
Schmp.: 129-130 °C

### Beispiel 27:

Komponente A: 5-Methoxy-1-phenylsulfonyl-1*H*-2-indol
Komponente B: 2,4-dimethoxy-benzoesäurechlorid
5-Methoxy-1-phenylsulfonyl-1*H*-2-indolyl(2,4-dimethoxyphenyl)-1-methanon
Schmp.: 62-64 °C

### Beispiel 27A:

Komponente A: 5-Methoxy-1-phenylsulfonyl-1H-2-indol
Komponente B: 3,4-Dimethoxybenzoesäurechlorid
5-Methoxy-1-phenylsulfonyl-1*H*-2-indolyl(3,4-dimethoxyphenyl)-1-methanon
Schmp.: 75 °C (Zers.)

### Beispiel 27B:

Komponente A: 5-Methoxy-1-phenylsulfonyl-1H-2-indol
Komponente B: 3,5-Dimethoxybenzoesäurechlorid
5-Methoxy-1-phenylsulfonyl-1*H*-2-indolyl(3,5-dimethoxyphenyl)-1-methanon
Schmp.: 122-123 °C

### Beispiel 28:

Komponente A: 1-Phenylsulfonyl-1*H*-2-indol
Komponente B: 3-pyridinyl-carbonsäurechlorid
1-Phenylsulfonyl-1*H*-2-indolyl(3-pyridinyl)-1-methanon
Schmp.: 124-125 °C

### Beispiel 29:

Komponente A: 5-Methoxy-1-phenylsulfonyl-1*H*-2-indol
Komponente B: 2-pyridinyl-carbonsäurechlorid
5-Methoxy-1-phenylsulfonyl-1*H*-2-indolyl(2-pyridinyl)-1-methanon
Schmp.: 207 °C

### Beispiel 30:

Komponente A: 4-(1-Phenylsulfonyl-1*H*-2-indol
Komponente B: 4-Cyano-benzoesäurechlorid
4-(1-Phenylsulfonyl-1*H*-2-indolylcarbonyl)-1-benzolcarbonitril
Schmp.: 175-177 °C

### Beispiel 31:

Komponente A: 2-Fluorphenyl(5-methoxy-1-phenylsulfonyl-1 H-2-indol)
Komponente B: 2-Fluor-benzoesäurechlorid
2-Fluorphenyl(5-methoxy-1-phenylsulfonyl-1*H*-2-indolyl)-1-methanon
Schmp.: 199-205 °C

### Beispiel 32:

Komponente A: 5-methoxy-1-phenylsulfonyl-1*H*-2-indol
Komponente B: 2,6-Difluor-benzoesäurechlorid
2,6-Difluorphenyl(5-methoxy-1-phenylsulfonyl-1*H*-2-indolyl)-1-methanon
Schmp.: 124 °C

### Beispiel 33:

Komponente A: 5-Methoxy-1-phenylsulfonyl-1*H*-2-indol
Komponente B: 2-Methyl-benzoesäurechlorid
5-Methoxy-1-phenylsulfonyl-1*H*-2-indolyl(2-methylphenyl)-1-methanon
Schmp.: 149-153 °C

### Beispiel 34:

Komponente A: 5-Methoxy-1-phenylsulfonyl-1*H*-2-indol
Komponente B: 3-trifluormethylphenyl-benzoesäurechlorid
5-Methoxy-1-phenylsulfonyl-1*H*-2-indolyl(3-trifluormethylphenyl)-1-methanon
Schmp.: 175-177 °C

### Beispiel 35:

Komponente A: 4-Fluorphenyl(5-methoxy-1-phenylsulfonyl-1*H*-2-indol
Komponente B: 4-Fluor-benzoesäurechlorid
4-Fluorphenyl(5-methoxy-1-phenylsulfonyl-1*H*-2-indolyl)-1-methanon
Schmp.: 123-128 °C

### Beispiel 36:

Komponente A: 5-Methoxy-1-phenylsulfonyl-1*H*-2-indol
Komponente B: 3,4-dichloro-benzoesäurechlorid
5-Methoxy-1-phenylsulfonyl-1*H*-2-indolyl(3,4-dichlorophenyl)-1-methanon
Schmp.: 141-144 °C

### Beispiel 37:

Komponente A: 5-Methoxy-1-phenylsulfonyl-1*H*-2-indol
Komponente B: 4-chloro-benzoesäurechlorid
5-Methoxy-1-phenylsulfonyl-1*H*-2-indolyl(4-chlorophenyl)-1-methanon
Schmp.: 146-148 °C

### Beispiel 38:

Komponente A: 5-Methoxy-1-phenylsulfonyl-1*H*-2-indol
Komponente B: 4-bromo-benzoesäurechlorid
5-Methoxy-1-phenylsulfonyl-1*H*-2-indolyl(4-bromophenyl)-1-methanon
Schmp.: 145-148 °C

### Beispiel 39:

Komponente A: 5-Methoxy-1-phenylsulfonyl-1*H*-2-indol
Komponente B: 3,4,5-trimethoxy-benzoesäurechlorid
5-Methoxy-1-phenylsulfonyl-1*H*-2-indolyl(3,4,5-trimethoxyphenyl)-1-methanon
Schmp.: 140-142 °C

### Beispiel 40:

Komponente A: 5-Methoxy-1-phenylsulfonyl-1*H*-2-indol
Komponente B: 4-pentyloxy-benzoesäurechlorid
5-Methoxy-1-phenylsulfonyl-1*H*-2-indolyl(4-pentyloxyphenyl)-1-methanon
Schmp.: 118-120 °C

### Beispiel 41:

Komponente A: 5-Methoxy-1-phenylsulfonyl-1*H*-2-indol
Komponente B: 1-Naphthyl-carbonsäurechlorid
5-Methoxy-1-phenylsulfonyl-1*H*-2-indolyl(1-naphthalenyl)-1-methanon
Schmp.: 225-228 °C

### Beispiel 42:

Komponente A: 5-methoxy-1-phenylsulfonyl-1*H*-2-indol
Komponente B: 4-tert-Buty-benzoesäurechlorid
4-*tert*-Butylphenyl(5-methoxy-1-phenylsulfonyl-1*H*-2-indolyl-1-methanon)
Schmp.: 161-163 °C

### Beispiel 43:

Komponente A: 5-Methoxy-1-phenylsulfonyl-1*H*-2-indol
Komponente B: 2,3-dimethoxy-benzoesäurechlorid
5-Methoxy-1-phenylsulfonyl-1*H*-2-indolyl(2,3-dimethoxyphenyl)-1-methanon
Schmp.: 128°C

### Beispiel 44:

Komponente A: 5-Methoxy-1-phenylsulfonyl-1*H*-2-indol
Komponente B: 2,3,4-trimethoxy-benzoesäurechlorid
5-Methoxy-1-phenylsulfonyl-1*H-*2-indolyl(2,3,4-trimethoxyphenyl)-1-methanon
Schmp.: 57-59 °C

### Beispiel 45:

Komponente A: 5-Methoxy-1-phenylsulfonyl-1*H*-2-indol
Komponente B: 4-methyl-benzoesäurechlorid
5-Methoxy-1-phenylsulfonyl-1*H*-2-indolyl(4-methylphenyl)-1-methanon
Schmp.: 126-127 °C

### Beispiel 46:

Komponente A: 5-Methoxy-1-phenylsulfonyl-1*H*-2-indol
Komponente B: 4-ethyl-benzoesäurechlorid
5-Methoxy-1-phenylsulfonyl-1*H*-2-indolyl(4-ethylphenyl)-1-methanon
Schmp.: 107-108 °C

### Beispiel 47:

Komponente A: 5-Methoxy-1-phenylsulfonyl-1*H*-2-indol
Komponente B: 4-propyl-benzoesäurechlorid
5-Methoxy-1-phenylsulfonyl-1*H*-2-indolyl(4-propylphenyl)-1-methanon
Schmp.: 112-114 °C

### Beispiel 48:

Komponente A: 5-Methoxy-1-phenylsulfonyl-1*H*-2-indol
Komponente B: 2-chloro-6-fluoro-benzoesäurechlorid
5-Methoxy-1-phenylsulfonyl-1 H-2-indolyl(2-chloro-6-fluorophenyl)-1-methanon
Schmp.: 130 °C

### Beispiel 49:

Komponente A: 5-Methoxy-1-phenylsulfonyl-1*H*-2-indol
Komponente B: 2,5-dimethyl-benzoesäurechlorid
5-Methoxy-1-phenylsulfonyl-1*H*-2-indolyl(2,5-dimethylphenyl)-1-methanon
Schmp.: 164 °C

### Beispiel 50:

Komponente A: 5-Methoxy-1-phenylsulfonyl-1*H*-2-indol
Komponente B: 2-nitro-benzoesäurechlorid
5-Methoxy-1-phenylsulfonyl-1*H*-2-indolyl(2-nitrophenyl)-1-methanon
Schmp.: 190-191 °C

### Beispiel 51:

Komponente A: 5-Methoxy-1-phenylsulfonyl-1*H*-2-indol
Komponente B: 2-amino-benzoesäurechlorid
5-Methoxy-1-phenylsulfonyl-1*H*-2-indolyl(2-aminophenyl)-1-methanon

### Beispiel 52:

Komponente A: 5-Methoxy-1-phenylsulfonyl-1*H*-2-indol
Komponente B: 3-nitro-benzoesäurechlorid
5-Methoxy-1-phenylsulfonyl-1*H*-2-indolyl(3-nitrophenyl)-1-methanon
Schmp.: 228-230 °C

### Beispiel 53:

Komponente A: 5-Methoxy-1-phenylsulfonyl-1*H*-2-indol
Komponente B: 3-amino-benzoesäurechlorid
5-Methoxy-1-phenylsulfonyl-1*H*-2-indolyl(3-aminophenyl)-1-methanon
Schmp.: 188-189 °C

### Beispiel 54:

Komponente A: 5-Methoxy-1-phenylsulfonyl-1*H*-2-indol
Komponente B: 4-nitro-benzoesäurechlorid
5-Methoxy-1-phenylsulfonyl-1*H*-2-indolyl(4-nitrophenyl)-1-methanon
Schmp.: 161-162 °C

### Beispiel 55:

Komponente A: 5-Methoxy-1-phenylsulfonyl-1*H*-2-indol
Komponente B: 4-amino-benzoesäurechlorid
5-Methoxy-1-phenylsulfonyl-1*H*-2-indolyl(4-aminophenyl)-1-methanon

### Beispiel 56:

Komponente A: 5-Methoxy-1-phenylsulfonyl-1*H*-2-indol
Komponente B: 3-methoxy-2-nitro-benzoesäurechlorid
5-Methoxy-1-phenylsulfonyl-1*H*-2-indolyl(3-methoxy-2-nitrophenyl)-1-methanon
Schmp.: 180 °C

### Beispiel 57:

Komponente A: 5-Methoxy-1-phenylsulfonyl-1*H*-2-indol
Komponente B: 2-amino-3-methoxy-benzoesäurechlorid
5-Methoxy-1-phenylsulfonyl-1*H*-2-indolyl(2-amino-3-methoxyphenyl)-1-methanon

### Beispiel 58:

Komponente A: 5-Methoxy-1-phenylsulfonyl-1*H*-2-indol
Komponente B: 2-methyl-3-nitro-benzoesäurechlorid
5-Methoxy-1-phenylsulfonyl-1*H*-2-indolyl(2-methyl-3-nitrophenyl)-1-methanon
Schmp.: 210-211 °C

### Beispiel 59:

Komponente A: 5-Methoxy-1-phenylsulfonyl-1*H*-2-indol
Komponente B: 3-amino-2-methyl-benzoesäurechlorid
5-Methoxy-1-phenylsulfonyl-1*H*-2-indolyl(3-amino-2-methylphenyl)-1-methanon
Schmp.: 206-207 °C

### Beispiel 60:

Komponente A: 5-methoxy-1-phenylsulfonyl-1*H*-2-indol
Komponente B: Cyclopropylcarbonsäurechlorid
Cyclopropyl(5-methoxy-1-phenylsulfonyl-1*H*-2-indolyl)-1-methanon
Schmp.: 118-120 °C

### Beispiel 61:

Komponente A: 5-methoxy-1-phenylsulfonyl-1*H*-2-indol
Komponente B: Cyclobutylcarbonsäurechlorid
Cyclobutyl(5-methoxy-1-phenylsulfonyl-1*H*-2-indolyl)-1-methanon
Schmp.: 146-147 °C

### Beispiel 62:

Komponente A: 5-Benzyloxy-1-phenylsulfonyl-1*H*-2-indol
Komponente B: Benzoesäurechlorid
5-Benzyloxy-1-phenylsulfonyl-1*H*-2-indolylphenyl-1-methanon
Schmp.: 205-207 °C

### Beispiel 63:

Komponente A: 5-Benzyloxy-1-phenylsulfonyl-1*H*-2-indol
Komponente B: 3-chloro-benzoesäurechlorid
5-Benzyloxy-1-phenylsulfonyl-1*H*-2-indolyl(3-chlorophenyl)-1-methanon
Schmp.: 150-152 °C

### Beispiel 64:

Komponente A: 5-Benzyloxy-1-phenylsulfonyl-1*H*-2-indol
Komponente B: 4-chloro-benzoesäurechlorid
5-Benzyloxy-1-phenylsulfonyl-1*H*-2-indolyl(4-chlorophenyl)-1-methanon
Schmp.: 63-65 °C

### Beispiel 65:

Komponente A: 5-Benzyloxy-1-phenylsulfonyl-1*H*-2-indol
Komponente B: 4-methoxy-benzoesäurechlorid
5-Benzyloxy-1-phenylsulfonyl-1*H*-2-indolyl(4-methoxyphenyl)-1-methanon
Schmp.: 70-72 °C

### Beispiel 66:

Komponente A: 5-Benzyloxy-1-phenylsulfonyl-1*H*-2-indol
Komponente B: 3,4,5-trimethoxy-benzoesäurechlorid
5-Benzyloxy-1-phenylsulfonyl-1*H*-2-indolyl(3,4,5-trimethoxyphenyl)-1-methanon
Schmp.: 150-152 °C

### Beispiel 67:

Komponente A: 5-Benzyloxy-1-phenylsulfonyl-1*H*-2-indol
Komponente B: 2-methoxy-benzoesäurechlorid
5-Benzyloxy-1-phenylsulfonyl-1*H*-2-indolyl-(2-methoxyphenyl)-1-methanon
Schmp.: 115-116°C

### Beispiel 68:

Komponente A: 5-Benzyloxy-1-phenylsulfonyl-1*H*-2-indol
Komponente B: 3-methoxy-benzoesäurechlorid
5-Benzyloxy-1-phenylsulfonyl-1*H*-2-indolyl-(3-methoxyphenyl)-1-methanon
Schmp.: 129-131 °C

### Beispiel 69:

Komponente A: 5-methoxy-1-phenylsulfonyl-1*H*-2-indol
Komponente B: 4-lsochinolyl-carbonsäurechlorid
4-Isochinolinyl(5-methoxy-1-phenylsulfonyl-1*H*-2-indolyl)-1-methanon
Schmp.: 189-190 °C

### Beispiel 70:

Komponente A: 5-methoxy-1-phenylsulfonyl-1*H*-2-indol
Komponente B: 1-Isochinolyl-carbonsäurechlorid
1-Isochinolinyl(5-methoxy-1-phenylsulfonyl-1*H*-2-indolyl)-1-methanon
Schmp.: 200 °C

### Beispiel 71:

Komponente A: 1-Phenylsulfonyl-1*H*-pyrrolo[2,3-*b*]pyridin
Komponente B: 2-methoxy-benzoesäurechlorid
1-Phenylsulfonyl-1*H*-pyrrolo[2,3-*b*]pyridin-2-yl(2-methoxyphenyl)-1-methanon
Schmp.:124-125 °C

### Beispiel 72:

Komponente A: 1-Phenylsulfonyl-1*H*-pyrrolo[2,3-*b*]pyridin
Komponente B: 3-methoxy-benzoesäurechlorid
1-Phenylsulfonyl-1*H*-pyrrolo[2,3-*b*]pyridin-2-yl(3-methoxyphenyl)-1-methanon
Schmp.:139-140 °C

### Beispiel 73:

Komponente A: 1-Phenylsulfonyl-1*H*-pyrrolo[2,3-*b*]pyridin
Komponente B: 3,4,5-trimethoxy-benzoesäurechlorid
1-Phenylsulfonyl-1*H*-pyrrolo[2,3-*b*]pyridin-2-yl(3,4,5-trimethoxyphenyl)-1-methanon
Schmp.:180-181 °C

### Beispiel 74:

Komponente A: 1-Phenylsulfonyl-1*H*-pyrrolo[2,3-*b*]pyridin
Komponente B: 2,4-dimethoxy-benzoesäurechlorid
1-Phenylsulfonyl-1*H*-pyrrolo[2,3-*b*]pyridin-2-yl(2,4-dimethoxyphenyl)-1-methanon
Schmp.:190-195 °C (Zers.) °C

### Beispiel 75:

Komponente A: 5-Methoxy-1-phenylsulfonyl-1*H*-pyrrolo[2,3-*b*]pyridin
Komponente B: 2-methoxy-benzoesäurechlorid
5-Methoxy-1-phenylsulfonyl-1*H*-pyrrolo[2,3-*b*]pyridin-2-yl(2-methoxyphenyl)-1-methanon

### Beispiel 76:

Komponente A: 5-Methoxy-1-phenylsulfonyl-1*H*-pyrrolo[2,3-*b*]pyridin
Komponente B: 3-methoxy-benzoesäurechlorid
5-Methoxy-1-phenylsulfonyl-1*H*-pyrrolo[2,3-*b*]pyridin-2-yl(3-methoxyphenyl)-1-methanon

### Beispiel 77:

Komponente A: 5-Methoxy-1-phenylsulfonyl-1*H*-pyrrolo[2,3-*b*]pyridin
Komponente B: 3,4,5-trimethoxy-benzoesäurechlorid
5-Methoxy-1-phenylsulfonyl-1*H*-pyrrolo[2,3-*b*]pyridin-2-yl(3,4,5-trimethoxyphenyl)-1-methanon

### Beispiel 78:

Komponente A: 5-Methoxy-1-phenylsulfonyl-1*H*-pyrrolo[2,3-*b*]pyridin
Komponente B: 2,4-dimethoxy-benzoesäurechlorid
5-Methoxy-1-phenylsulfonyl-1*H*-pyrrolo[2,3-b]pyridin-2-yl(2,4-dimethoxyphenyl)-1-methanon

### Beispiel 79:

Komponente A: 5-Methoxy-1-phenylsulfonyl-1*H*-pyrrolo[3,2-*b*]pyridin
Komponente B: Benzoesäurechlorid
5-Methoxy-1-phenylsulfonyl-1*H*-pyrrolo[3,2-*b*]pyridin-2-ylphenyl-1-methanon

### Beispiel 80:

Komponente A: 5-Methoxy-1-phenylsulfonyl-1*H*-pyrrolo[3,2-*b*]pyridin
Komponente B: 2-methoxy-benzoesäurechlorid
5-Methoxy-1-phenylsulfonyl-1*H*-pyrrolo[3,2-*b*]pyridin-2-yl(2-methoxyphenyl)-1-methanon

### Beispiel 81:

Komponente A: 5-Methoxy-1-phenylsulfonyl-1*H*-pyrrolo[2,3-*c*]pyridin
Komponente B:3-methoxy-benzoesäurechlorid
5-Methoxy-1-phenylsulfonyl-1*H*-pyrrolo[2,3-*c*]pyridin-2-yl(3-methoxyphenyl)-1-methanon

### Beispiel 82:

Komponente A: 5-Methoxy-1-phenylsulfonyl-1H-pyrrolo[2,3-c]pyridin
Komponente B: 2,4-dimethoxy-benzoesäurechlorid
5-Methoxy-1-phenylsulfonyl-1*H*-pyrrolo[2,3-c]pyridin-2-yl(2,4-dimethoxyphenyl)-1-methanon

### Beispiel 83:

Komponente A: 5-Methoxy-1-phenylsulfonyl-1*H*-pyrrolo[2,3-*c*]pyridin
Komponente B: 3,4,5-trimethoxy-benzoesäurechlorid
5-Methoxy-1-phenylsulfonyl-1*H*-pyrrolo[2,3-*c*]pyridin-2-yl(3,4,5-trimethoxyphenyl)-1-methanon

### Beispiel 84:

Komponente A: 5-Methoxy-1-phenylsulfonyl-1*H*-pyrrolo[3,2-*b*]pyridin
Komponente B: 2-methoxy-benzoesäurechlorid
5-Methoxy-1-phenylsulfonyl-1*H*-pyrrolo[3,2-*b*]pyridin-2-yl(2-rnethoxyphenyl-1-methanone
Schmp.: 197-198 °C

### Beispiel 85:

Komponente A: 5-Methoxy-1-phenylsulfonyl-1*H*-pyrrolo[3,2-*b*]pyridin
Komponente B: 3-methoxy-benzoesäurechlorid
5-Methoxy-1-phenylsulfonyl-1*H*-pyrrolo[3,2-*b*]pyridin-2-yl(3-methoxyphenyl-1-methanone
Schmp.: 147-149 °C

### Beispiel 86:

Komponente A: 5-Methoxy-1-phenylsulfonyl-1*H*-pyrrolo[3,2-*b*]pyridin
Komponente B: 2,4-dimethoxy-benzoesäurechlorid
5-Methoxy-1-phenylsulfonyl-1*H*-pyrrolo[3,2-*b*]pyridin-2-yl(2,4-dimethoxyphenyl-1-methanone
Schmp.: 132 °C

### Beispiel 87:

Komponente A: 5-Methoxy-1-phenylsulfonyl-1*H*-pyrrolo[3,2-*b*]pyridin
Komponente B: 3,4,5-trimethoxy-benzoesäurechlorid
5-Methoxy-1-phenylsulfonyl-1*H*-pyrrolo[3,2-*b*]pyridin-2-yl(3,4,5-trimethoxyphenyl-1-methanone
Schmp.: 190-191 °C

### Allgemeine Vorschriften zur Herstellung der erfindungsgemäß verwendeten 1H-2-indolylphenyl-1-methanone

Methode A: Das entsprechende N-geschützte Methanon-Derivat (Ausgangskomponente) (1.8 mmol) wird in einer Mischung aus 10 % Natriumhydroxid (20 ml) und Ethanol (40 ml) 2 bis 15 Stunden zum Rückfluß erhitzt (DC-Kontrolle). Nach Abkühlen auf Raumtemperatur wird die Lösung auf 100 ml Wasser gegossen und mit Ethylacetat extrahiert. Die organische Phase wird über Natriumsulfat getrocknet und das Lösungsmittel entfernt. Das Rohprodukt wird aus Ethylacetat umkristallisiert.
Methode B: Eine Mischung des entsprechenden N-geschützten MethanonDerivats (Ausgangskomponente) (1.8 mmol) und 0.79 g (2.5 mmol) Tetrabutylammoniumfluorid Trihydrat wird in 20 ml THF/Methanol 1:1 zum Rückfluß erhitzt. Nach Reaktionsende (30 min - 4 Stunden, DC-Kontrolle) wird abgekühlt und die Mischung auf 100 ml Wasser gegossen. Es wird mit Ethylacetat extrahiert und die organische Phase über Natriumsulfat getrocknet. Das Lösungsmittel wird langsam eingeengt, bis das Produkt auszukristallisieren beginnt.

### Beispiel 88:

Ausgangskomponente: Verbindung gemäß Beispiel 10
Methode A oder B
1*H*-2-Indolylphenyl-1-methanon
Schmp.: 145-147 °C

### Beispiel 89

Ausgangskomponente: Verbindung gemäß Beispiel 11
Methode A oder B
1*H*-2-Indolyl(2-methoxyphenyl)-1-methanon
Schmp.: 129-130 °C

### Beispiel 90:

Ausgangskomponente: Verbindung gemäß Beispiel 12
Methode A oder B
1*H*-2-Indolyl(3-methoxyphenyl)-1-methanon
Schmp.: 124-126 °C

### Beispiel 91:

Ausgangskomponente: Verbindung gemäß Beispiel 13
Methode A oder B
1*H*-2-Indolyl(2,4-dimethoxyphenyl)-1-methanon
Schmp.: 134-135 °C

### Beispiel 92:

Ausgangskomponente: Verbindung gemäß Beispiel 14
Methode A oder B
1*H*-2-Indolyl(3,4,5-trimethoxyphenyl)-1-methanon
Schmp.: 148-150 °C

### Beispiel 93:

Ausgangskomponente: Verbindung gemäß Beispiel 15
Methode A oder B
3-Methyl-1*H*-2-indolyl(2-methoxyphenyl)-1-methanon
Schmp.: 152-153 °C

### Beispiel 94:

Ausgangskomponente: Verbindung gemäß Beispiel 16
Methode A oder B
3-Methyl-1*H*-2-indolyl(3-methoxyphenyl)-1-methanon
Schmp.: 131 °C

### Beispiel 95:

Ausgangskomponente: Verbindung gemäß Beispiel 17
Methode A oder B
3-Methyl-1*H*-2-indolyl(2,4-dimethoxyphenyl)-1-methanon
Schmp.: 124-126 °C

### Beispiel 96:

Ausgangskomponente: Verbindung gemäß Beispiel 18
Methode A oder B
3-Methyl-1*H*-2-indolyl(3,4,5-trimethoxyphenyl)-1-methanon
Schmp.: 138-144 °C

### Beispiel 97:

Ausgangskomponente: Verbindung gemäß Beispiel 19
Methode A oder B
5-Methyl-1*H*-2-indolyl(2-methoxyphenyl)-1-methanon
Schmp.: 165-167 °C

### Beispiel 98:

Ausgangskomponente: Verbindung gemäß Beispiel 20
Methode A oder B
5-Methyl-1*H*-2-indolyl(3-methoxyphenyl)-1-methanon
Schmp.: 192-202 °C

### Beispiel 99:

Ausgangskomponente: Verbindung gemäß Beispiel 21
Methode A oder B
5-Methyl-1 *H*-2-indolyl(2,4-dimethoxyphenyl)-1-methanon

### Beispiel 99A:

Ausgangskomponente: Verbindung gemäß Beispiel XX
Methode A oder B
5-Methoxy-1*H*-2-indolyl(3,4-dimethoxyphenyl)-1-methanon
Schmp.: 187°C

### Beispiel 99B:

Ausgangskomponente: Verbindung gemäß Beispiel YY
Methode A oder B
5-Methoxy-1*H*-2-indolyl(3, 5-dimethoxyphenyl)-1-methanon
Schmp.: 141-142 °C

### Beispiel 100:

Ausgangskomponente: Verbindung gemäß Beispiel 22
Methode A oder B
5-Methyl-1*H*-2-indolyl(3,4,5-trimethoxyphenyl)-1-methanon
Schmp.: 202-203 °C

### Beispiel 101:

Ausgangskomponente: Verbindung gemäß Beispiel 23
Methode A oder B
5-Methoxy-1*H*-2-indolylphenyl-1-methanon
Schmp.: 162 °C

### Beispiel 102:

Ausgangskomponente: Verbindung gemäß Beispiel 24
Methode A oder B
5-Methoxy-1*H*-2-indolyl(2-methoxyphenyl)-1-methanon
Schmp.: 127 °C

### Beispiel 103:

Ausgangskomponente: Verbindung gemäß Beispiel 25
Methode A oder B
5-Methoxy-1*H*-2-indolyl(3-methoxyphenyl)-1-methanon
Schmp.: 147-148 °C

### Beispiel 104:

Ausgangskomponente: Verbindung gemäß Beispiel 26
Methode A oder B
5-Methoxy-1*H*-2-indolyl(4-methoxyphenyl)-1-methanon
Schmp.: 165 °C

### Beispiel 105:

Ausgangskomponente: Verbindung gemäß Beispiel 27
Methode A oder B
5-Methoxy-1*H*-2-indolyl(2,4-dimethoxyphenyl)-1-methanon
Schmp.: 160-161 °C

### Beispiel 106:

Ausgangskomponente: Verbindung gemäß Beispiel 29
Methode A oder B
5-Methoxy-1*H*-2-indolyl(2-pyridinyl)-1-methanon
Schmp.: 201 °C

### Beispiel 107:

Ausgangskomponente: Verbindung gemäß Beispiel 30 (?)
Methode A oder B
4-(1*H*-2-Indolylcarbonyl)-1-benzolcarbonsäure
Schmp.: > 220 °C.

### Beispiel 108:

Ausgangskomponente: Verbindung gemäß Beispiel 31
Methode A oder B
2-Fluorphenyl(5-methoxy-1*H*-2-indolyl)-1-methanon
Schmp.: 145 °C

### Beispiel 109:

Ausgangskomponente: Verbindung gemäß Beispiel (?)
Methode A oder B
5-Methoxy-1-phenylsulfonyl-1*H*-2-indolyl(3-trifluormethylphenyl)-1-methanon
Schmp.: 165°C

### Beispiel 110:

Ausgangskomponente: Verbindung gemäß Beispiel 33
Methode A oder B
5-Methoxy-1*H*-2-indolyl(2-methylphenyl)-1-methanon
Schmp.: 120 °C

### Beispiel 111:

Ausgangskomponente: Verbindung gemäß Beispiel 34
Methode A oder B
5-Methoxy-1*H*-2-indolyl(3-trifluormethylphenyl)-1-methanon
Schmp.: 193-195 °C

### Beispiel 112:

Ausgangskomponente: Verbindung gemäß Beispiel 35
Methode A oder B
4-Fluorphenyl(5-methoxy-1*H*-2-indolyl)-1-methanon
Schmp.: 168 °C

### Beispiel 113:

Ausgangskomponente: Verbindung gemäß Beispiel 36
Methode A oder B
5-Methoxy-1*H*-2-indolyl(3,4-dichlorophenyl)-1-methanon
Schmp.: 190-192 °C

### Beispiel 114:

Ausgangskomponente: Verbindung gemäß Beispiel 37
Methode A oder B
5-Methoxy-1*H*-2-indolyl(4-chlorophenyl)-1-methanon
Schmp.: 191-193 °C

### Beispiel 115:

Ausgangskomponente: Verbindung gemäß Beispiel 38
Methode A oder B
5-Methoxy-1*H*-2-indolyl(4-bromophenyl)-1-methanon
Schmp.: 188-190 °C

### Beispiel 116:

Ausgangskomponente: Verbindung gemäß Beispiel 39
Methode A oder B
5-Methoxy-1*H*-2-indolyl(3,4,5-trimethoxyphenyl)-1-methanon
Schmp.: 210-211 °C

### Beispiel 117:

Ausgangskomponente: Verbindung gemäß Beispiel 40
Methode A oder B
5-Methoxy-1*H*-2-indolyl(4-pentyloxyphenyl)-1-methanon
Schmp.: 139-141 °C

### Beispiel 118:

Ausgangskomponente: Verbindung gemäß Beispiel 41
Methode A oder B
5-Methoxy-1*H*-2-indolyl(1-naphthalenyl)-1-methanon
Schmp.: 174-175 °C

### Beispiel 119:

Ausgangskomponente: Verbindung gemäß Beispiel 42
Methode A oder B
4-*tert*-Butylphenyl(5-methoxy-1*H*-2-indolyl-1-methanon)
Schmp.: 204-207 °C

### Beispiel 120:

Ausgangskomponente: Verbindung gemäß Beispiel 43
Methode A oder B
5-Methoxy-1*H*-2-indolyl(2,3-dimethoxyphenyl)-1-methanon

### Beispiel 121:

Ausgangskomponente: Verbindung gemäß Beispiel 44
Methode A oder B
5-Methoxy-1*H*-2-indolyl(2,3,4-trimethoxyphenyl)-1-methanon
Schmp.: 156°C

### Beispiel 122:

Ausgangskomponente: Verbindung gemäß Beispiel 45
Methode A oder B
5-Methoxy-1*H*-2-indolyl(4-methylphenyl)-1-methanon
Schmp.: 200 °C

### Beispiel 123:

Ausgangskomponente: Verbindung gemäß Beispiel 46
Methode A oder B
5-Methoxy-1*H*-2-indolyl(4-ethylphenyl)-1-methanon
Schmp.: 154-155 °C

### Beispiel 124:

Ausgangskomponente: Verbindung gemäß Beispiel 47
Methode A oder B
5-Methoxy-1*H*-2-indolyl(4-propylphenyl)-1-methanon
Schmp.: 145-146 °C

### Beispiel 125:

Ausgangskomponente: Verbindung gemäß Beispiel 48
Methode A oder B
5-Methoxy-1*H*-2-indolyl(2-chloro-6-fluorophenyl)-1-methanon
Schmp.: 168-170 °C

### Beispiel 126:

Ausgangskomponente: Verbindung gemäß Beispiel 49
Methode A oder B
5-Methoxy-1*H*-2-indolyl(2,5-dimethylphenyl)-1-methanon
Schmp.: 152-153 °C

### Beispiel 127:

Ausgangskomponente: Verbindung gemäß Beispiel 50
Methode A oder B
5-Methoxy-1*H*-2-indolyl(2-nitrophenyl)-1-methanon
Schmp.: 185-187 °C

### Beispiel 128:

Ausgangskomponente: Verbindung gemäß Beispiel 51
Methode A oder B
5-Methoxy-1*H*-2-indolyl(2-aminophenyl)-1-methanon
Schmp.: 144-145 °C

### Beispiel 129:

Ausgangskomponente: Verbindung gemäß Beispiel 52
Methode A oder B
5-Methoxy-1*H*-2-indolyl(3-nitrophenyl)-1-methanon
Schmp.: 221-222 °C

### Beispiel 130:

Ausgangskomponente: Verbindung gemäß Beispiel 53
Methode A oder B
5-Methoxy-1*H*-2-indolyl(3-aminophenyl)-1-methanon

### Beispiel 131:

Ausgangskomponente: Verbindung gemäß Beispiel 54
Methode A oder B
5-Methoxy-1*H*-2-indolyl(4-nitrophenyl)-1-methanon

### Beispiel 132:

Ausgangskomponente: Verbindung gemäß Beispiel 55
Methode A oder B
5-Methoxy-1*H*-2-indolyl(4-aminophenyl)-1-methanon

### Beispiel 133:

Ausgangskomponente: Verbindung gemäß Beispiel 56
Methode A oder B
5-Methoxy-1*H*-2-indolyl(3-methoxy-2-nitrophenyl)-1-methanon
Schmp.: 212 °C (Zers.)

### Beispiel 134:

Ausgangskomponente: Verbindung gemäß Beispiel 57
Methode A oder B
5-Methoxy-1*H*-2-indolyl(2-amino-3-methoxyphenyl)-1-methanon

### Beispiel 135:

Ausgangskomponente: Verbindung gemäß Beispiel 58
Methode A oder B
5-Methoxy-1*H*-2-indolyl(2-methyl-3-nitrophenyl)-1-methanon
Schmp.: 199-200 °C

### Beispiel 136:

Ausgangskomponente: Verbindung gemäß Beispiel 59
Methode A oder B
5-Methoxy-1*H*-2-indolyl(3-amino-2-methylphenyl)-1-methanon
Schmp.: 163-165 °C

### Beispiel 137:

Ausgangskomponente: Verbindung gemäß Beispiel 60
Methode A oder B
Cyclopropyl(5-methoxy-1*H*-2-indolyl)-1-methanon
Schmp.: 205-207 °C

### Beispiel 138:

Ausgangskomponente: Verbindung gemäß Beispiel 61
Methode A oder B
Cyclobutyl(5-methoxy-1*H*-2-indolyl)-1-methanon
Schmp.: 175-179 °C

### Beispiel 139:

Ausgangskomponente: Verbindung gemäß Beispiel 62
Methode A oder B
5-Benzyloxy-1*H*-2-indolylphenyl-1-methanon
Schmp.: 187-188 °C

### Beispiel 140:

Ausgangskomponente: Verbindung gemäß Beispiel 63
Methode A oder B
5-Benzyloxy-1*H*-2-indolyl(3-chlorophenyl)-1-methanon
Schmp.: 163-165 °C

### Beispiel 141:

Ausgangskomponente: Verbindung gemäß Beispiel 64
Methode A oder B
5-Benzyloxy-1*H*-2-indolyl(4-chlorophenyl)-1-methanon
Schmp.: 188-190 °C

### Beispiel 142:

Ausgangskomponente: Verbindung gemäß Beispiel 65
Methode A oder B
5-Benzyloxy-1*H*-2-indolyl(4-methoxyphenyl)-1-methanon
Schmp.: 155-157 °C

### Beispiel 143:

Ausgangskomponente: Verbindung gemäß Beispiel 66
Methode A oder B
5-Benzyloxy-1*H*-2-indolyl(3,4,5-trimethoxyphenyl)-1-methanon
Schmp.: 165-167 °C

### Beispiel 144:

Ausgangskomponente: Verbindung gemäß Beispiel 67
Methode A oder B
5-Benzyloxy-1*H*-2-indolyl-(2-methoxyphenyl)-1-methanon
Schmp.: 150-151 °C

### Beispiel 145:

Ausgangskomponente: Verbindung gemäß Beispiel 68
Methode A oder B
5-Benzyloxy-1*H*-2-indolyl-(3-methoxyphenyl)-1-methanon
Schmp.: 153-154 °C

### Beispiel 146:

Ausgangskomponente: Verbindung gemäß Beispiel 69
Methode A oder B
4-Isoquinolinyl(5-methoxy-1*H*-2-indolyl)-1-methanon
Schmp.: 228-230 °C

### Beispiel 147:

Ausgangskomponente: Verbindung gemäß Beispiel 70
Methode A oder B
1-Isoquinolinyl(5-methoxy-1*H*-2-indolyl)-1-methanon
Schmp.: 175 °C

### Beispiel 148:

Ausgangskomponente: Verbindung gemäß Beispiel 71
Methode A oder B
1*H*-Pyrrolo[2,3-*b*]pyridin-2-yl(2-methoxyphenyl)-1-methanon
Schmp.:211-213 °C

### Beispiel 149:

Ausgangskomponente: Verbindung gemäß Beispiel 72
Methode A oder B
1*H*-Pyrrolo[2,3-*b*]pyridin-2-yl(3-methoxyphenyl)-1-methanon
Schmp.: 166-168 °C

### Beispiel 150:

Ausgangskomponente: Verbindung gemäß Beispiel 73
Methode A oder B
1*H*-Pyrrolo[2,3-*b*]pyridin-2-yl(3,4,5-trimethoxyphenyl)-1-methanon
Schmp. :205-206 °

### Beispiel 151:

Ausgangskomponente: Verbindung gemäß Beispiel 74
Methode A oder B
1*H*-Pyrrolo[2,3-*b*]pyridin-2-yl(2,4-dimethoxyphenyl)-1-methanon
Schmp.:208-210 °C (Zers.)

### Beispiel 152:

Ausgangskomponente: Verbindung gemäß Beispiel 75
Methode A oder B
5-Methoxy-1*H*-pyrrolo[2,3-*b*]pyridin-2-yl(2-methoxyphenyl)-1-methanon

### Beispiel 153:

Ausgangskomponente: Verbindung gemäß Beispiel 76
Methode A oder B
5-Methoxy-1*H*-pyrrolo[2,3-*b*]pyridin-2-yl(3-methoxyphenyl)-1-methanon

### Beispiel 154:

Ausgangskomponente: Verbindung gemäß Beispiel 77
Methode A oder B
5-Methoxy-1*H*-pyrrolo[2,3-*b*]pyridin-2-yl(3,4,5-trimethoxyphenyl)-1-methanon

### Beispiel 155:

Ausgangskomponente: Verbindung gemäß Beispiel 78
Methode A oder B
5-Methoxy-1*H*-pyrrolo[2,3-*b*]pyridin-2-yl(2,4-dimethoxyphenyl)-1-methanon

### Beispiel 156:

Ausgangskomponente: Verbindung gemäß Beispiel 79
Methode A oder B
5-Methoxy-1*H*-pyrrolo[3,2-*b*]pyridin-2-ylphenyl-1-methanon

### Beispiel 157:

Ausgangskomponente: Verbindung gemäß Beispiel 80
Methode A oder B
5-Methoxy-1*H*-pyrrolo[3,2-*b*]pyridin-2-yl(2-methoxyphenyl)-1-methanon

### Beispiel 158:

Ausgangskomponente: Verbindung gemäß Beispiel 81
Methode A oder B
5-Methoxy-1*H*-pyrrolo[2,3-*c*]pyridin-2-yl(3-methoxyphenyl)-1-methanon

### Beispiel 159:

Ausgangskomponente: Verbindung gemäß Beispiel 82
Methode A oder B
5-Methoxy-1*H*-pyrrolo[2,3-*c*]pyridin-2-yl(2.4-dimethoxyphenyl)-1-methanon

### Beispiel 160:

Ausgangskomponente: Verbindung gemäß Beispiel 83
Methode A oder B
5-Methoxy-1*H*-pyrrolo[2,3-*c*]pyridin-2-yl(3,4,5-trimethoxyphenyl)-1-methanon

### Beispiel 161:

Ausgangskomponente: Verbindung gemäß Beispiel 84
Methode A oder B
5-Methoxy-1*H*-pyrrolo[3,2-*b*]pyridin-2-yl(2-methoxyphenyl-1-methanone
Schmp.: 190 °C

### Beispiel 162:

Ausgangskomponente: Verbindung gemäß Beispiel 85
Methode A oder B
5-Methoxy-1*H*-pyrrolo[3,2-*b*]pyridin-2-yl(3-methoxyphenyl-1-methanone
Schmp.: 150 °C

### Beispiel 163:

Ausgangskomponente: Verbindung gemäß Beispiel 86
Methode A oder B
5-Methoxy-1*H*-pyrrolo[3,2-*b*]pyridin-2-yl(2,4-dimethoxyphenyl-1-methanone
Schmp.: 100 °C (Zers.)

### Beispiel 164:

Ausgangskomponente: Verbindung gemäß Beispiel 87
Methode A oder B
5-Methoxy-1*H*-pyrrolo[3,2-*b*]pyridin-2-yl(3,4,5-trimethoxyphenyl-1-methanone
Schmp.: 233 °C

Alternativ können die erfindungsgemäß verwendeten Verbindungen auch durch Umsetzung eines N-geschützten substituierten Indolderivats mit einer entsprechenden Nitrilverbindung gemäß dem nachstehenden Vorschriftsbeispiel hergestellt werden.

### Beispiel 147 (nach alternativem Verfahren hergestellt):

### Verbindung: 1-Isoquinolinyl(5-methoxy-1H-2-indolyl)-1-methanon

Zu einer auf -78 °C gekühlten Lösung von 1-(tert.-Butyloxycarbonyl)-5-methoxyindol (5 mmol) in 10 ml trockenem THF tropfte man n-Butyllithium (5.5 mmol, 1.6 M in Hexan, Aldrich). Nach 30 Minuten bei -78 °C tropfte man eine Lösung von 1-Cyanoisochinolin (7.5 mmol), in 2 ml THF gelöst, langsam zu. Man ließ über Nacht langsam auf Raumtemperatur erwärmen (16 Stunden). Zur dunkelbraunen Lösung fügte man 50 ml einer Mischung aus Trifluoressigsäure:Dichlormethan = 4:1, rührte 90 Minuten bei Raumtemperatur, extrahierte mit 30 ml Dichlormethan, wusch die organische Phase mit Wasser, gesättigter Kaliumcarbonatlösung und wieder Wasser (je 20 ml) und entfernte das Lösungsmittel im Vakuum. Das resultierende braune Öl wurde in 10 ml Ethanol aufgeschlämmt und auf 300 ml Eiswasser gegossen. Der grün-braune Niederschlag wurde durch Filtration isoliert und säulenchromatografisch unter Normaldruck an Kieselgel 60 (Laufmittel Diethylether:Hexan = 1:1) gereinigt. Ausbeute: 160 mg (10 %), gelbe Nadeln

### Allgemeine Vorschrift zur Herstellung von N-Oxiden der Azaindole und deren Derivatisierung

### Herstellung der N-Oxide:

1.00 mmol des Pyridinderivats werden in 20 ml Dichlormethan bei 0 °C mit 2 mmol meta-Chlorperbenzoesäure versetzt. Man läßt auf R.T. erwärmen und rührt 24 h bei dieser Temp. Nach Zugabe von 10 ml konz. NaHCO₃-Lsg. Wird die org. Phase abgetrennt und die wässrige Phase 10 mal mit je 25 ml Dichlormethan extrahiert. Die vereinigten org. Phasen werden über MgSO₄ getrocknet und das Lösungsmittel entfernt. Der verbleibende Rückstend wird mit wenig Diethylether versetzt, wobei das Produkt als pulveriger Niederschlag erhalten wird (Ausb.: 65 %).

### Beispiel 164:

Ausgangskomponente: Verbindung gemäß Beispiel 150
1*H*-pyrrolo[2,3-*b*]pyridin-N-oxid-2-yl(3,4,5-trimethoxyphenyl)-1-methanon
Schmp.: 90 bis 92 °C

### Umsetzung der N-Oxide mit Acetanhydrid:

0.5 mmol des N-Oxids wird mit 15 ml Essigsäureanhydrid versetzt. Nach Zugabe von einem Tropfen Wasser wird 12 h refluxiert. Sobald das Edukt laut DC-Kontrolle abreagiert hat wird das Lösungsmittel abgezogen und der Rückstand mit wenig Dichlormethan aufgenommen und mit NaHCO₃-Lösung gewaschen. Entfernen des Lösungsmittels und Versetzen des Rückstands mit Diethylether lierfert das Produkt als pulverigen Niederschlag (60 %)

### Beispiel 165:

Ausgangskomponente: Verbindung gemäß Beispiel XXX
6-[2-(3,4,5-Trimethoxybenzoyl)-1-acetyl-1*H*-pyrrolo[2,3*b*]pyridin]ethanoat
Schmp.: 151-152°C

### Allgemeine Vorschrift zur Herstellung der erfindungsgemäß verwendeten N-substituierte 1H-2-Indolylphenyl-1-methanone

Eine Mischung des entsprechenden 1*H*-2-Indolylphenyl-1-methanons (Ausgangsprodukt) (5.0 mol), dem Hydrochlorid des entsprechenden Aminoalkylchlorids (15.0 mmol) und 40.0 mmol Kaliumcarbonat wird in 50 ml abs. Aceton 14 Stunden zum Rückfluß erhitzt. Nach dem Abkühlen wird das Reaktionsgemisch auf 250 ml Wasser gegossen und mit Dichlormethan extrahiert. Die organische Phase wird über Natriumsulfat getrocknet. Nach Entfernen des Lösungsmittels wird der Rückstand durch Säulenchromatographie gereinigt.

### Beispiel 166:

Ausgangsprodukt gemäß Beispiel 101
5-Methoxy-1-(2-dimethylaminoethyl)-1*H*-2-indolylphenyl-1-methanon
Schmp.: 38-40 °C

### Beispiel 167:

Ausgangsprodukt gemäß Beispiel 101
5-Methoxy-1-(3-dimethylaminopropyl)-1*H*-2-indolylphenyl-1-methanon
Schmp.: 51-52 °C

### Beispiel 168:

Ausgangsprodukt gemäß Beispiel 101
5-Methoxy-1-(2-pyrrolidinoethyt)-1*H*-2-indolylphenyl-1-methanon
Schmp.: 68-71 °C

### Beispiel 169:

Ausgangsprodukt gemäß Beispiel 101
5-Methoxy-1-(2-piperidinoethyl)-1*H*-2-indolylphenyl-1-methanon
Schmp.: 55-57 °C

### Beispiel 170:

Ausgangsprodukt gemäß Beispiel 101
5-Methoxy-1-(2-morpholinoethyl)-1*H*-2-indolylphenyl-1-methanon
Schmp.: 66-68 °C

### Beispiel 171:

Ausgangsprodukt gemäß Beispiel 101
5-Methoxy-1-(2-phenylmethyloxyethyl)-1*H*-2-indolylphenyl-1-methanon
Schmp.: 95-97 °C

Weitere Beispiele sind:

### Beispiel 172:

3-Ethoxy-5-methoxy-1*H*-2-indolyl(2-nitrophenyl)-1-methanon

### Beispiel 173:

5-methoxy-1*H*-2-indolyl(2-thienyl)-1-methanon

### Beispiel 174:

5-methoxy-1*H*-2-indolyl(3-fluor-phenyl)-1-methanon

### Beispiel 175:

5-methoxy-1*H*-2-indolyl(3-trifluormethoxy-phenyl)-1-methanon

### Beispiel 176:

5-methoxy-1*H*-2-indolyl(3-difluormethylthio-phenyl)-1-methanon

### Beispiel 177:

5-methoxy-1*H*-2-indolyl(3-hydroxy-phenyl)-1-methanon

### Beispiel 178:

5-methoxy-1*H*-2-indolyl(3-butanoyloxyphenyl)-1-methanon

### Ergebnisse der pharmakologischen Testung

Die in-vitro Testung in selektierten Tumormodellen ergab die nachfolgenden pharmakologischen Aktivitäten.

### Beispiel 1: Antitumorwirkung

Die Substanzen D-64131 (Bsp.101), D-68143 (Bsp. 102), D-68144 (Bsp. 103), D-68150 (Bsp. 116) und D-68172 (Bsp. 105) wurden in einem Proliferationstest an etablierten Tumorzellinien auf ihre anti-proliferative Aktivität hin untersucht. Der verwendete Test bestimmt die zelluläre Dehydrogenase-Aktivität und ermöglicht eine Bestimmung der Zellvitalität und indirekt der Zellzahl. Bei den verwendeten Zellinien handelt es sich um die humanen Glioma Zellinien A-172 (ATCC CRL-1620), U118 (ATCC HTB-15) und U373 (ATCC HTB-17), die Ratten Glioma Zellinie C6 (ATCC CCL107) sowie die humane Zervixkarzinom Zellinie KB /HeLa (ATCC CCL17). Es handelt sich hierbei um sehr gut charakterisierte, etablierte Zellinien, die von ATCC erhalten und in Kultur genommen wurden.

Die in Tab. 1 und Abb. 1 zusammengefaßten Ergebnisse zeigen eine sehr potente antitumoraktive Wirkung der genannten Substanzen. Hervorzuheben ist eine Konzentrationsabhängige Wirkung, die zu vergleichbaren maximalen Hemmungen führt. Dabei konnten definierte Wirksamkeiten bestimmt werden: D-68144 > D-68150 ≥ D-64131 + D-68143> D-68172 (zunehmende antitumoraktive Potenz von D-68172 zu D-68144). Diese Abfolge der Wirksamkeit war bei allen untersuchten Zellinien zu beobachten und ist als ein Hinweis auf einen definierten molekularen Wirkmechanismus zu bewerten.

### Tabelle 1.

Antitumoraktive Potenz verschiedener Derivate im XTT Zytotoxizitätstest an den Glioma Zellinien C6, A-172, U118, U373 sowie der Zervixkarzinom Zellinie HeLa/KB. Angegeben ist die IC₅₀ aus Konzentrations-Wirkungsversuchen in nM.

In Klammern ist die Anzahl der unabhängigen Versuche angegeben, sofern die Versuche mehrfach durchgeführt wurden.

| Beispiel | Code-Nr | C6 | A-172 | U118 | U373 | KB/Hela |
|---|---|---|---|---|---|---|
| 101 | D-64131 | 96.5 (2) | 51 | 24 | 22 | 24 (2) |
| 102 | D-68143 | 98 (2) | 73 | 28 | 29 | 35 (2) |
| 103 | D-68144 | 9.6 (2) | 15 | 8.3 | 5.0 | 6.6 |
| 116 | D-68150 | 77 | 18.5 (2) | 19.4 | 19.7 | 32 |
| 105 | D-68172 | 180 | 330 (2) | 119 (2) | 75 (2) | 107 (2) |

In Fig. 1 ist dargestellt:
Graphische Darstellung der Konzentrations-abhängigen antitumoralen Aktivität verschiedener Derivate im XTT Zytotoxizitätstest an der KB / HeLa Zervixkarzinom Zellinie.

### Beispiel 2: Zellzyklusanalyse mittels Fluoreszenz-aktivierter Zellsortierung

Die Substanzen D-64131 (Bsp. 101), D-68144 (Bsp.103) und D-68150 (Bsp. 116) wurden an der humanen Glioblastomzellinie U373 mittels Floureszenz-aktivierter Zellsortierung (FACS) weiter untersucht. Die gewählte Methode ermöglicht den Nachweis einer Zellzyklus-spezifischen Substanzwirkung. Hierzu wurde über Messung des DNA-Gehaltes der Anteil der Zellen in den Phasen G1, S, G2 und M des Zellzyklus bestimmt. In Abb. 2 ist das Ergebnis dieser Analyse zusammengefaßt. Dargestellt ist der Anteil an Zellen in der Metaphase der mitotischen Teilung (M Phase des Zellzyklus; 2N Chromosomen). Eindeutig nachweisbar ist für alle getesteten Substanzen eine Konzentrations-abhängige Arretierung der Zellen in der Mitose, die mit der in Tabelle 1 und Abb. 1 gezeigten anti-proliferativen Wirkung korreliert. Die Substanzen führen somit über eine Hemmung der Zellteilung zu einer Wachstumsarretierung, die nachfolgend zu einem Absterben der Tumorzellen (Apoptose) führt.

In Fig. 2 ist dargestellt:
Zellzyklusanalyse von Substanz behandelten U373 Gliomazellen mittels FACS. Dargestellt ist der prozentuale Anteil an Zellen mit 2N Chromosomen, d.h. Zellen in der Metaphase der mitotischen Zellteilung, als Funktion der Substanzkonzentration.

### Vergleich der biologischen Aktivität der erfindungsgemäß verwendeten Verbindung D-68144 (Beispiel 103) mit den Verbindungen 1d/4d gemäß der Publikation von Medarde et al.

In der Publikation von M. Medarde et al. 1998, Eur. J. Med. Chem. Vol.33, S.71-77 werden Combretastatin Analoge beschrieben, die in einem Proliferationsassay an den Tumorzellinien P388 (Leukämie, murin), A549 (Lungenkarzinom, human), HT29 (Kolonkarzinom, human) und Mel28 (Melanom, human) eine anti-tumorale Wirkung aufweisen. Das verwendete Testsystem ist dem vorstehend beschriebenen Testsystem vergleichbar. Genannte Tumorzellen werden für 72h mit den Substanzen behandelt und die Zellzahl direkt (P388) bzw. indirekt über Kristallvioletfärbung (Mel28, A549. HT29) bestimmt. Die bekannte Verbindung 1-Methyl-2-(3,4,5-trimethoxyphenyl)carbonylmethyl-indol (Verbindung 4d) zeigt in diesem Test eine inhibitorische Aktivität von IC₅₀ = 0.3 bis 0.6µM, die bekannte Verbindung 1-Methyl-3-(3-hydroxy-4-methoxyphenyl)carbonylmethyl-indol (Verbindung 1 d) eine inhibitorische Aktivität von IC₅₀ = 3.6 bis 8.9 µM. Im Gegensatz hierzu zeigt die erfindungsgemäße Verbindung D-68144 eine inhibitorische Aktivität an diversen Gliomalinien von IC₅₀ = 0.005 bis 0.015 µM. Überraschenderweise ist die erfindungsgemäße Verbindung D-68144 um den Faktor 40-60 aktiver als die in der Publikation von Medarde et al. beschriebene Verbindung 4d.

### Beschreibung der verwendeten Methoden.

### XTT-Test auf zelluläre Dehydrogenase-Aktivität

Die adherent wachsenden Tumorzellinien C6, A-172, U118, U373 und HeLa/KB wurden unter Standardbedingungen im Begasungsbrutschrank bei 37°C, 5% CO₂ und 95% Luftfeuchtigkeit kultiviert. Am Versuchstag 1 werden die Zellen mit Trypsin / EDTA abgelöst und durch Zentrifugation pelletiert. Nachfolgend wird das Zellpellet in dem jeweiligen Kulturmedium in der entsprechenden Zellzahl resuspendiert und in eine 96-well Mikrotiterplatte umgesetzt. Die Platten werden dann über Nacht im Begasungsbrutschrank kultiviert. Die Testsubstanzen werden als 10mM Stammlösungen in DMSO angesetzt und am Versuchstag 2 mit Kulturmedium In den entsprechenden Konzentrationen verdünnt. Die Substanzen in Kulturmedium werden dann zu den Zellen gegeben und für 45h im Begasungsbrutschrank inkubiert. Als Kontrolle dienen Zellen, die nicht mit Testsubstanz behandelt werden. Für das XTT-Assay werden 1mg/ml XTT (Natrium 3'-[1-(phenylaminocarbonyl)-3,4-tetrazolium]-bis(4-methoxy-6-nitro) benzensulfonsäure) in RPMI-1640 Medium ohne Phenolrot gelöst. Zusätzlich wird eine 0,383 mg/ml PMS (N-Methyl Dibenzopyrazine Methylsulfat) Lösung in Phosphat-gepufferter Salzlösung (PBS) hergestellt, Am Versuchstag 4 wird auf die Zellplatten, die inzwischen 45 h mit den Testsubstanzen inkubiert wurden, 75µl/well XTT-PMS-Mischung pipettiert. Dazu wird kurz vor Gebrauch die XTT-Lösung mit der PMS-Lösung im Verhältnis 50:1 (Vol:Vol) gemischt. Anschließend werden die Zellplatten im Begasungsbrutschrank für weitere 3h inkubiert und Im Photometer die optische Dichte (OD₄₉₀ₙₘ) bestimmt.
Mittels der bestimmten OD₄₉₀ₙₘ wird die prozentuale Hemmung relativ zur Kontrolle berechnet und in Form einer Konzentrations-Wirkungskurve halblogarithmisch aufgetragen. Die IC₅₀ wird mittels einer Regressionsanalyse aus der Konzentrations-Wirkungskurve mit dem Programm Graphpad berechnet.

### Zellzyklusanalyse mittels FACS

U373 Gliomazellen werden in adherenter, subkonfluenter Kultur für 24h mit Substanz behandelt, nachfolgend abgelöst und 1x mit PBS gewaschen. Insgesamt 5x10⁶ Zellen / Meßpunkt werden in 1ml 80% Methanol (-20°C) fixiert, für 30min auf Eis gestellt und bei 4°C gelagert. Für die FACS Analyse werden die Zellen in PBS mit 0.1% Saponin, 20µg/ml Propidiumjodid und 1mg/ml RNAse A für 30min bei 37°C inkubiert. Nach waschen in PBS/Saponin Puffer werden die Zellen auf einem Calibur Durchflußzytometer (Becton Dickinson) analysiert.

Die in-vitro Testung in selektierten Tumormodellen zeigte die nachfolgenden pharmakologischen Aktivitäten.

### Beispiel 3: Tubulin-inhibitorische und zytotoxische Aktivität ausgewählter Verbindungen

Ausgewählte Verbindungen wurden in einem in-vitro Test auf Hemmung der Polymerisation von Rinderhirn getestet. In diesem Test wird durch Zyklen von Polymerisation und Depolymerisation aufgereinigtes Tubulin eingesetzt, welches durch Zugabe von GTP und Erwärmung zur Polymerisation gebracht wird, In Tabelle 1 sind die IC₅₀ Werte der Polymerisationshemmung von Tubulin angegeben. Als Referenzsubstanzen sind Vincristin und Colchicin als bekannte Tubulininhibitoren mit einbezogen. Als sehr potente Inhibitoren sind beispielsweise D-70316 und D-81187 mit IC₅₀ Werten von 0.81 und 0.39µM hervorzuheben.

In Tabelle 1 sind außerdem die zytotoxischen bzw. wachstumshemmenden Aktivitäten der an der humanen Zervixkarzinom Zellinie HeLa/KB getesteten Verbindungen angegeben. Hier zeigen sich einige der Verbindungen als hochzytotoxische Wirkstoffe. Hervorzuheben sind beispielsweise D-64131, D-68144, D-70316 und D-81187.

**Tabelle 1**

| Hemmung der Tubulinpolymerisation und zytotoxische Aktivität an der HeLa/KB Zervixkarzinomzellinie für ausgewählte Verbindungen. Die Zytotoxizität bzw. Wachstumshemmung ist in IC₅₀ in der Konzentration µg/ml oder nM angegeben. | | | |
|---|---|---|---|
| **Beispiel** | **Struktur** | Tubulinhemmung /IC₅₀ [µM] | **Zytotoxizität KB/HeLa IC50 [µg/ml]** |
| **D-65499** (Bsp.115) | 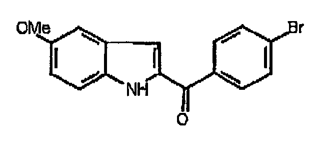 | 4.3 (2) | ~0.3 |
| **D-65500** (Bsp.114) | 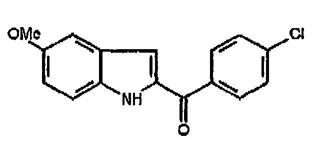 | 4.98 (2) | ~1.0 |
| **D-65502** (Bsp. 170) | 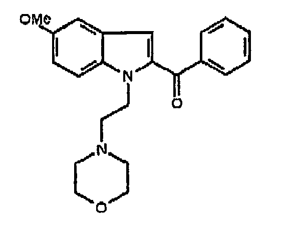 | 5.58 (2) | ~0.5 |
| **D-64131** (Bsp. 101) | 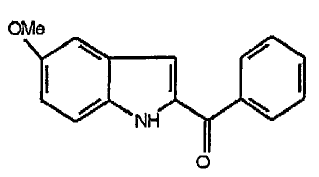 | 2.2 | 24nM (2) |
| **D-68143** (Bsp. 102) | 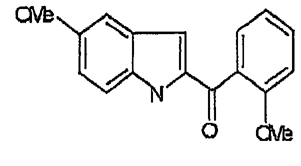 | 2.12 (3) | 35nM (2) |
| **D-68144** (Bsp. 103) | 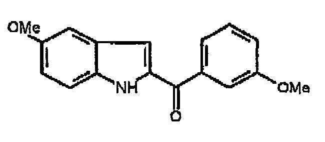 | 2.42 (2) | 6.6nM |
| **D-68150** (Bsp. 116) | 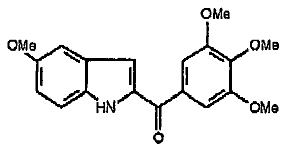 | 10 (1) | 32nM |
| **D-68172** (Bsp. 105) | 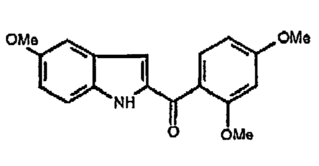 | <10(1) | 107nM (2) |
| **D-68213** (Bsp. 118) | 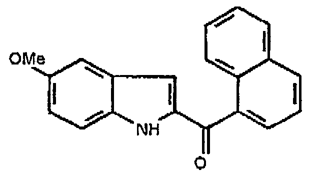 | 2.93(2) | ~0.1 |
| **D-68887** (Bsp. 122) | 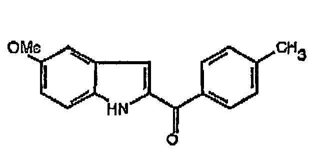 | 1.5 (2) | ~0.3 |
| **D-68888** (Bsp. 108) | 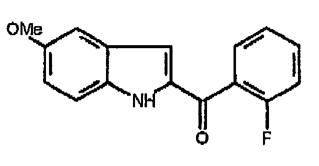 | 1.03 (2) | ~0.2 |
| **D-68901** (Bsp. 111) | 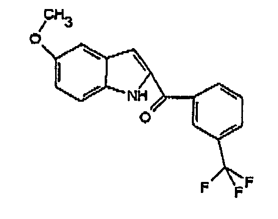 | 1.46(2) | ~0.01 |
| **D-68906** (Bsp. 126) | 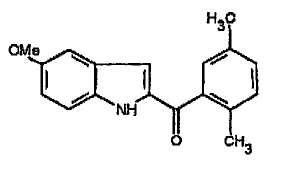 | 5.19 (2) | ~0.2 |
| **D-70026** (Bsp. 113) | 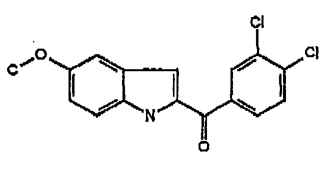 | 2.49(2) | ~0.1 |
| **D-70038** (Bsp. 110) | 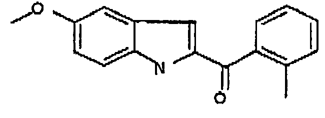 | 4.84 (2) | ~0.5 |
| **D-70046** (Bsp. 172) | 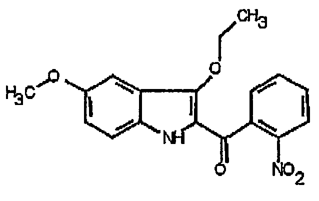 | 2.44 (2) | ~2 |
| **D-70047** (Bsp. 128) | 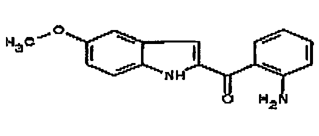 | 6.68 (2) | ~2 |
| **D-70048** (Bsp. 129) | 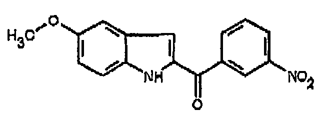 | 0.85 (2) | ~0.03 |
| **D-70261** (Bsp. 103) | 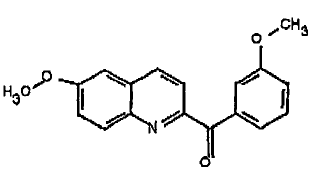 | 3.2 (2) | n.b. |
| **D-70288** (Bsp. 100) | 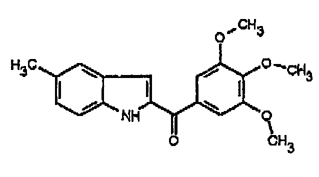 | 0.86 (2) | ~0.01 |
| **D-70289** (Bsp. 97) | 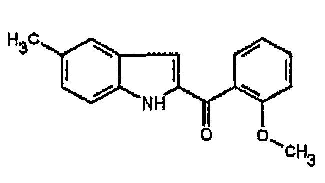 | 3.54 (2) | ~0.1 |
| **D-70316** (Bsp. 99B) | 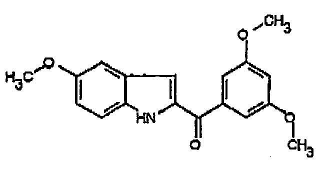 | 0.81 (2) | 18.6nM (2) |
| **D-70438** (Bsp. 173) | 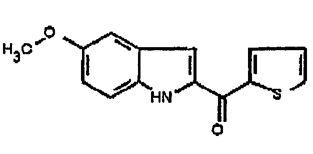 | 2.7 (2) | n.b. |
| **D-81167** (Bsp. 130) | 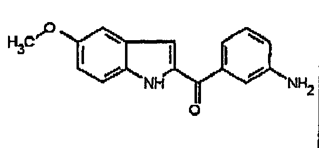 | 0.99 (2) | 0.089 |
| **D-81187** (Bsp. 174) | 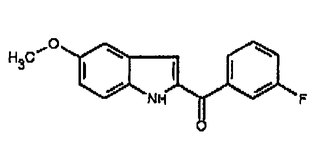 | 0.39 (2) | 19.7nM (2) |
| **D-81194** (Bsp. 175) | 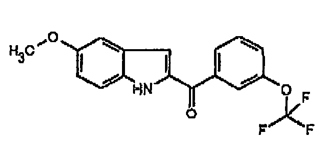 | 1.74 (2) | 0.086 |
| **D-81196** (Bsp. 176) | 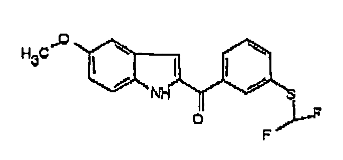 | 2,05 (2) | 0,134 |
| **D-81755** (Bsp. 177) | 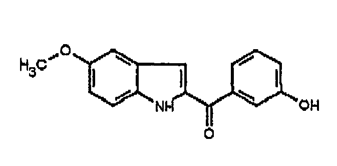 | 0.66 (2) | 0.063 |
| **D-81756** (Bsp. 178) | 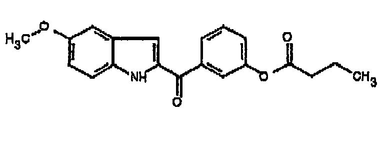 | 0.85 (2) | 0.093 |
| **Vincristin** | 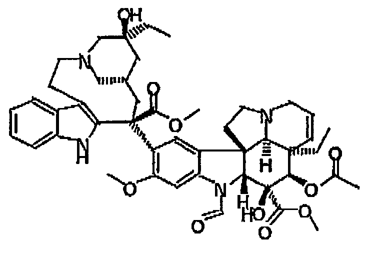 | 0.09 (3) | 1,5nM (2) |
| **Colchicin** | 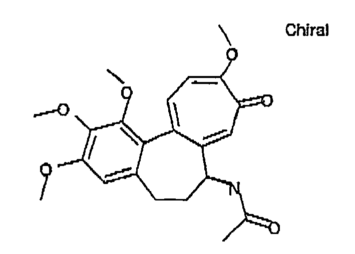 | 1.0 (2) | 18.7nM (2) |

### Beispiel 4 Zellzyklus spezifische Wirkung im RKOp21 Modell

Als ein Modell zur Untersuchung der Zellzyklus-spezifischen Wirkung wurde das RKOp21 Zellsystem verwendet (M.Schmidt et al. Oncogene 19(20):2423-9, 2000). Bei RKO handelt es sich um eine humane Kolonkarzinomlinie, in der der Zellzyklusinhibitor p21^{waf1} mittels des Ecdyson Expressionssystems induziert zur Expression gebracht und zu einem Zellzyklusarrest spezifisch in G1 und G2 führt. Eine unspezifisch wirkende Substanz hemmt die Proliferation unabhängig davon, ob die RKO Zelle in G1 oder G2 arretiert ist oder nicht. Zellzyklus-spezifische Substanzen wie beispielsweise Tubulininhibitoren sind hingegen nur dann zytotoxisch, wenn Zellen nicht arretiert sind und der Zellzyklus durchlaufen wird. In Tabelle 2 ist die zytotoxische bzw. wachstumshemmende Aktivität ausgewählter Verbindungen mit / ohne Expression von p21^{waf1}gezeigt. Alle getesteten Verbindungen zeigten im induzierten Zustand von p21^{waf1} eine geringe oder keine zytotoxische Aktivität. Dies unterstreicht die bereits in den FACS Analysen bestimmte Arretierung im Zellzyklus in G2/M und eine Zellzyklus spezifische Wirkung der untersuchten Verbindungen.

**Tabelle 2**

| Zytotoxische Aktivität ausgewählter Verbindungen im RKO p21^{waf1} Zellsystem. | | |
|---|---|---|
| **Substanz** | **RKO p21 induziert** | **RKO p21 Induziert IC₅₀ [nM]** |
| D-64131 | n.b. | 15(1) |
| D-68143 | n.b. | 28(1) |
| D-68144 | n.b. | 3.6(1) |
| D-68150 | n.b. | 16.8(1) |
| D-68172 | n.b. | 136 (1) |
| D-70316 | n.b. | 17.95 (2) |
| D-81187 | n.b. | 16.8(2) |
| Taxol | n.b. | 0.0078 µg/ml |

| | | |
|---|---|---|
| n.b. IC₅₀ nicht berechenbar | | |

### Beispiel 5 Aktivität von D-64131 in humanen Tumorxenograft Experimenten in Nacktmäusen

Für die in-vivo Experimente an Nacktmäusen wurden subkutan transplantierte Tumorfragmente des humanen Melanoms MEXF 989 bzw. des Rhabdomyosarkoms SXF 463 verwendet. D-64131 wurde in den Dosen 100 und 200mg/kg (Vehikel 10% DMSO in PBS / Tween 80 0.05%) über 2 Wochen (5 Applikationen je Woche; Montag bis Freitag) oral appliziert. In Experimenten mit beiden Tumoren zeigte sich eine sehr gute Wirksamkeit von D-64131. In dem Modell MEXF989 konnte eine Wachstumsinhibition von 81 % (200mg/kg/Tag) bzw. 66% (100mg/kg/Tag) erzielt werden. In dem Modell SXF463 zeigte die höhere Dosis von 200mg/kg eine 83% Wachstumsinhibition relativ zur Kontrolle. Diese Ergebnisse zeigen neben einer oralen Bioverfügbarkeit und sehr guten Verträglichkeit eine potente anti-Tumoraktivität in zwei humanen Turnorxenograftmodellen.

### Beschreibung der verwendeten Methoden.

### Rindertubulin Polymerisationsassay

Das Assay verwendet Tubulin isoliert durch Zyklen von Polymerisation und Depolymerisation aus Rinderhirn. Zuerst werden 85µl Mix bestehend aus 80µl PEM Puffer pH 6.6 (0.1 M Pipes, 1 mM EGTA,1 mM MgSO₄ p.H6.6) und 5µl 20mM GTP Stammlösung pro well in die Filterplatte Typ MultiScreen (0.22µM hydrophilic, low protein binding-Durapore Membrane, Fa. Millipore) vorgelegt. Dazu wird die Testsubstanz, gelöst in 100% DMSO, in der entsprechenden Menge pipettiert. Danach erfolgt die Zugabe von 10µl aufgereinigtem Rindertubulin (je well 50-60µg Tubulin). Die Filterplatte wird bei Raumtemperatur 20min mit 400rpm geschüttelt und nachfolgend 50µl / well Färbelösung (45% MeOH, 10% Essigsäure, 0.1% Naphthol Blue Black / Sigma) pipettiert. Nach einer Inkubationszeit von 2 Minuten wird die Färbelösung abgesaugt (Eppendorf Event 4160) und nachfolgend zweimal mit einer 90% Methanol / 2% Essigsäure Lösung gewaschen. Abschließend werden 200µl / weil Entfärbelösung (25mM NaOH, 50% Ethanol, 0.05mM EDTA) pipettiert. Nach einer Inkubation von 20 Minuten bei Raumtemperatur auf einem Schüttler (400rpm), wird im Photometer bei einer Absorption von 600 nM gemessen. Berechnet wird die prozentuale Hemmung bezogen auf den 100% Wert einer Positivkontrolle (keine Testsubstanz enthalten) bzw. der lC₅₀ Wert bei Aufnahme einer Konzentrationswirkungskurve.

### XTT-Test auf zelluläre Dehydrogenase-Aktivität

Ergänzend zu der Tumorzellinie HeLa/KB (siehe Tabelle 1) können für die Proliferationsexperimente die Zellinien C6, A-172, U118, U373, SKOV3 (ATCC HTB 77, humanes Ovarialadenokarzinom), SF268 (NCl 503138, humanes Gliom), NC1460 (NCI 503473; humanes nicht kleinzelliges Lungenkarzinom), MCF-7 (ATCC HTB22; humanes Mamma-adenokarzinom) und RKO (humanes Kolonadenokarzinom) verwendet werden.

### Zellzyklusanalyse mittels des RKOp21 Modelles

Das Assay wird in 96-Well Platten durchgeführt. Durch induzierbare Expression von p21^{waf1} werden die Zellen vollständig wachstumsarretiert, sterben aber nicht ab. Durch Vergleich der Wirksamkeit auf induzierte und nicht induzierte Zellen lassen sich Rückschlüsse auf den Wirkmechanismus (Zellzyklus-Spezifität) der Therapeutika ziehen. Nicht induzierte Zellen werden in etwa vierfach höherer Zellzahl ausgesät, da keine Teilung mehr während des Assays im Vergleich zu uninduzierten Zellen erfolgt (2x10⁴ Zellen/Well induziert, ca. 0.6x10⁴ Zellen / Well nicht induziert). Die Kontrollen sind unbehandelte Zellen (+/- Induktion). Die Induktion erfolgt mit 3µM Muristeron A. Am 1. Tag werden die Zellen ausgesetzt (+/- Muristeron A) und für 24h bei 37°C inkubiert. Am Tag 2 wird die Testsubstanz zugegeben (Kontrolle DMSO) und für weitere 48h bei 37°C inkubiert, bevor ein Standard XTT Assay durchgeführt wird.

### Orale Bioverfügbarkeit von D-64131:

D-64131 wurde zunächst in vitro an 12 permanenten humanen Tumorzellinien auf seine Antitumoraktivität untersucht. Die Zelllinien umfaßten Darm- (2), Magen- (1), Lunge- (3), Brust- (2), Melanom- (2), Ovarien- (1), Nieren (1) und Uterus- (1) Tumorzelllinien. Der mittlere IC50 von D-64131 über alle untersuchten Zelllinien unter Verwendung eines auf Propidiumiodid basierenden Zytotoxitätsassays betrug 0,34 µM. Melanom-, Darm- und Nieren-Tumorzellen waren dabei am empfindlichsten (IC50 = 4 nM). Für die untersuchten Lungen- und Magentumorzelllinien war der IC50 etwa 4 µM. D-64131 wirkte dabei als zellzyklus-spezifischer Wirkstoff durch Wechselwirkung mit Tubulin. D-64131 inhibierte die Polymerisation von Kälberhirntubulin mit einem IC50 von 2,2 µM. Die maximale tolerierte Dosis bei intraperitonealer (i.p.) Injektion bei Nacktmäusen war 400 mg/kg bei wöchentlicher Verabreichung. Zur peroralen (p.o.) Verabreichung wurden 100 und 200 mg/kg D-64131 mit der Dosierung "Qdx5" (1x täglich für 5 aufeinandertolgende Tage) über 2 Wochen verabreicht. Beide p. o. Dosierungen wurden sehr gut vertragen und zeigten keine Anzeichen von Toxizität oder Körpergewichtsverlust. Das letztere Dosierungsschema wurde zur Testung der Wirksamkeit von D-64131 im humanen Melanom-Xenograftmodel MEXF 989 verwendet. Die orale Behandlung mit D-64131 führte zu einer 81%igen Wachstumsinhibierung gegenüber Kontrolle bei 200 mg/kg/d und 66 % Wachstumsinhibierung bei 100 mg/kg/d. Im Rhabdomyosarcoma-Xenograftmodel SXF 463 wurde eine 83 %ige Wachstumsinhibierung bei 200 mg/kg/d gefunden. Die gefundenen Daten belegen, daß die erfindungsgemäßen Indol-Verbindungen potente cytotoxische Wirkstoffe darstellen, die in Zellzyklusspezifischer Weise durch Interferenz mit dem mitotischen Spindelapparat wirken. Hervorzuheben ist weiterhin die orale Bioverfügbarkeit der erfindungsgemäßen Indolverbindungen.

Basierend auf der gefundenen Wirksamkeit und Verträglichkeit des oral bioverfügbaren niedermolekularen Tubulininhibitors D-64131 ist dieser ein Kandidat für weitergehende klinische Prüfungen in den Phasen I und II. Nachstehend sind Beispiele für pharmazeutische Zubereitungen der erfindungsgemäßen Indolverbindungen und deren Herstellung aufgeführt.

### Beispiel I

### Tablette mit 50 mg Wirkstoff

### Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| Summe: | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt.

### Beispiel II

### Kapsel mit 50 mg Wirkstoff

### Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| Summe: | 160,0 mg |

### Herstellung:

(1) wird mit (3) verrieben. Diese Verreibung wird der Mischung aus (2) und (4) unter intensiver Mischung zugegeben. Diese Pulvermischung wird auf einer Kapselabfüllmaschine in Hartgelatine-Steckkapseln Größe 3 abgefüllt.

## Patentansprüche

1. Verwendung von mindestens einer Verbindung der allgemeinen Formel I 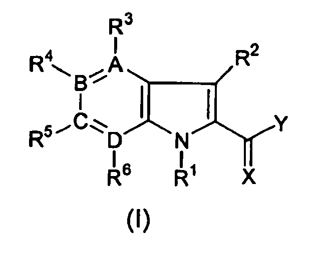 worin
R1 Wasserstoff, (C1-C6)-Alkylcarbonyl, vorzugsweise Acetyl, (C₁-C₆)-Alkyl, Mono-(C₁-C₆)-Alkylamino-(C₁-C₄)-alkyl, Di-(C₁-C₆)-Alkylamino-(C₁-C₄)-alkyl, wobei die beiden (C₁-C₆)-Alkylreste miteinander einen Ring bilden können, welcher gegenbenenfalls ein oder mehrere NH, N-(C1-C6)-Alkyl, O oder S-Glieder aufweist, (C6-C14)-Aryl-(C1-C6)-alkyl oder (C6-C14)-Aryl-(C1-C6)-alkoxy-(C1-C6)-alkyl bedeutet;
R2 ein Wasserstoffatom, Halogen, Cyano, Nitro, (C1-C6)-Alkyl, mit ein oder mehreren Halogenatomen substituiertes (C1-C6)-Alkyl, vorzugsweise Trifluormethyl, mit ein oder mehreren Halogenatomen substituiertes (C1-C6)-Alkoxy, vorzugsweise Trifluormethoxy, (C2-C6)-Alkenyl, (C2-C6)-Alkinyl, (C3-C8)-Cycloalkyl, (C1-C6)-Alkoxy, (C1-C6)-Alkoxycarbonyloxy, (C1-C6)-Alkylcarbonyloxy, (C1-C4)-Alkylthio, (C1-C4)-Alkylsulfinyl, (C1-C4)-Alkylsulfonyl, (C1-C6)-Alkoxy-(C1-C6)-alkyl, Amino, Mono-(C1-C6)-Alkylamino, Di-N,N-(C1-C6)-alkyl-amino, wobei die beiden (C1-C6)-Alkylreste miteinander einen Ring bilden können, welcher gegenbenenfalls ein oder mehrere NH, N-(C1-C6)-Alkyl, O oder S aufweist, (C6-C14)-Aryl, (C6-C14)-Aryloxy, (C6-C14)-Aryl-(C1-C4)-alkyl, (C6-C14)-Aryl-(C1-C4)-alkoxy-(C1-C4)-alkyl, (C1-C6)-alkylcarbonyl, (C1-C6)-Alkoxycarbonyl, oder Hydroxyl bedeutet;
A, B, C, D unabhängig voneinander für ein Stickstoffatom (wobei R3, R4, R5 und R6 dann für das freie Elektronenpaar am Stickstoffatom stehen) oder ein mit einem der Reste R3-R6 substituiertes Kohlenstoffatom stehen;
R3, R4, R5 und R6 unabhängig voneinander, wenn an Stickstoff gebunden, ein freies Elektronenpaar, oder, wenn an Kohlenstoff gebunden, Wasserstoff, Halogen, Cyano, Nitro, geradkettig oder verzweigtes (C1-C6)-Alkyl, mit einem oder mehreren Halogenatomen substituiertes geradkettig oder verzweigtes (C1-C6)-Alkyl, vorzugsweise Trifluormethyl, mit einem oder mehreren Halogenatomen substituiertes geradkettig oder verzweigtes (C1-C6)-Alkoxy, vorzugsweweise Trifluormethoxy, (C2-C6)-Alkenyl, (C2-C6)-Alkinyl, (C3-C8)-Cycloalkyl, geradkettig oder verzweigtes (C1-C6)-Alkoxy, vorzugsweise Methoxy, geradkettig oder verzweigtes (C1-C6)-Alkylendioxy, vorzugsweise Methylendioxy, (C1-C6)-Alkoxycarbonyloxy, (C1-C6)-Alkylcarbonyloxy, (C1-C4)-Alkylthio, (C1-C4)-Alkylsulfinyl, (C1-C4)-Alkylsulfonyl, Carboxy, Carboxy-(C1-C6)-alkylester, Carboxamid, N-(C1-C4)-Alkyl-carboxamid, N,N-di-(C1-C4)-alkyl-carboxamid, (C1-C6)-Alkoxy-(C1-C6)-alkyl, Amino, Mono-(C1-C6)-Alkylamino, N,N-di-(C1-C6)-alkyl-amino wobei die beiden C1-C6-Alkylreste miteinander einen Ring bilden können, welcher gegenbenenfalls ein oder mehrere NH, N-(C1-C6)-Alkyl, O oder S aufweist, (C6-C14)-Aryl, (C6-C14)-Aryloxy, (C6-C14)-Aryl-(C1-C4)-alkyl, (C6-C14)-Aryl-(C1-C4)-alkoxy-(C1-C4)-alkyl, (C1-C6)-Alkylcarbonyl, (C1-C6)-Alkylcarbonyloxy, (C1-C6)-Alkoxycarbonyl, Hydroxyl, wobei zwei direkt benachbarte Reste miteinander verbunden sein können, bedeuten;
Y unsubstituiertes oder ganz oder teilweise gleich oder verschieden substituiertes (C6-C14)-Aryl, vorzugsweise Phenyl oder 1- oder 2-Naphthyl, oder unsubstituiertes oder ganz oder teilweise gleich oder verschieden mindestens ein bis vier N, NH, N-(C1-C6)-alkyl, O und/oder S als Ringglieder aufweisendes (C1-C13)-Heteroaryl oder unsubstituiertes oder ganz oder teilweise gleich oder verschieden substituiertes (C3-C8)-Cycloalkyl, wobei die gleichen oder verschiedenen Substituenten unabhängig voneinander ausgewählt sind aus der Gruppe bestehend aus Halogen, vorzugsweise Fluor, Chlor, Brom oder Jod; Cyano; geradkettig oder verzweigtes Cyano-(C1-C6)-alkyl; Hydroxy; mit ein oder mehreren Hydroxy substituiertes geradkettig oder verzweigtes (C1-C6)-Alkyl; Carboxy; Carboxy-(C1-C6)-alkylester, Carboxamid; N-(C1-C6)-Alkyl-carboxamid, N,N-di-(C1-C4)-alkyl-carboxamid, Nitro, geradkettig oder verzweigtes (C1-C6)-Alkyl, mit einem oder mehreren Halogenatomen substituiertes geradkettig oder verzweigtes (C1-C6)-Alkyl, vorzugsweise Trifluormethyl, mit einem oder mehreren Halogenatomen substituiertes geradkettig oder verzweigtes (C1-C6)-Alkoxy, vorzugsweise Trifluormethoxy, geradkettig oder verzweigtes (C2-C6)-Alkenyl, geradkettig oder verzweigtes (C2-C6)-Alkinyl, (C3-C8)-Cycloalkyl, geradkettig oder verzweigtes (C1-C6)-Alkoxy, vorzugsweise Methoxy, geradkettig oder verzweigtes (C1-C6)-Alkylendioxy, vorzugsweise Methylendioxy, Thio (-SH), geradkettig oder verzweigtes (C1-C6)-Alkylthio, (C1-C6)-Alkylsulfinyl, (C1-C6)-Alkylsulfonyl, (C1-C6)-Alkoxy-(C1-C6)-alkyl, Amino, geradkettig oder verzweigtes Mono-(C1-C6)-Alkylamino, geradkettig oder verzweigtes N,N-di-(C1-C6)-alkyl-amino wobei die beiden (C1-C6)-Alkylreste miteinander einen Ring bilden können, welcher gegenbenenfalls ein oder mehrere NH, N-(C1-C6)-Alkyl, O und/oder S aufweisen kann, (C6-C14)-Aryl, (C6-C14)-Aryloxy, (C6-C14)-Aryl-(C1-C6)-alkyl, (C6-C14)-Aryl-(C1-C6)-alkoxy-(C1-C6)-alkyl, (C1-C6)-Alkylcarbonyl, (C1-C6)-Alkylcarbonyloxy, (C1-C6)-Alkoxycarbonyl, (C1-C6)-Alkoxycarbonyloxy, geradkettig oder verzweigtes Mono- und N,N-di-(C1-C6)-Alkylcarbonyl-amino, geradkettig oder verzweigtes Mono- und N,N-di-(C1-C6)-Alkoxycarbonylamino, geradkettig oder verzweigtes N-(C1-C6)-alkylcarbonyl-N-(C1-C6)-alkyl-amino, geradkettig oder verzweigtes N-(C1-C6)-alkoxycarbonyl-N-(C1-C6)-alkyl-amino, Formylamino, Formyl, wobei zwei direkt benachbarte Reste miteinander verbunden sein können, bedeutet;
X für ein Sauerstoff- oder Schwefelatom, für NH, oder für eine geminal (am gleichen C-Atom) substituiertes Hydroxy und Wasserstoff (-CH(OH)-)steht;
deren Stereoisomere, deren Tautomere, deren Gemische sowie die pharmazeutisch verträglichen Salze davon, zur Herstellung eines Arzneimittels zur Behandlung von Tumorerkrankungen bei Säugetieren, vorzugsweise beim Menschen.

2. Verwendung von mindestens einer Verbindung der allgemeinen Formel I nach Anspruch 1, **dadurch gekennzeichnet, daß** R1-R6, A, B, C, D, X und Y die in Anspruch 1 genannte Bedeutung haben, mit der Maßgabe, daß mindestens einer der Reste R3-R6 für geradkettig oder verzweigtes (C1-C6)-Alkoxy, vorzugsweise Methoxy; geradkettig oder verzweigtes (C1-C6)-Alkyl, vorzugsweise Methyl; geradkettig oder verzweigtes (C1-C6)-Alkylendioxy, vorzugsweise Methylendioxy, Hydroxyl; mit einem oder mehreren Halogenatomen substituiertes geradkettig oder verzweigtes (C1-C6)-Alkoxy, vorzugsweise Trifluormethoxy; mit einem oder mehreren Halogenatomen substituiertes geradkettig oder verzweigtes (C1-C6)-Alkyl, vorzugsweise Trifluormethyl; steht.

3. Verwendung von mindestens einer Verbindung der allgemeinen Formel I nach Anspruch 1 oder 2, **dadurch gekennzeichnet, daß** daß R1, R2, R3, R5, R6, A, B, C, D, X und Y die vorstehend genannte Bedeutung haben und der Rest R4 für geradkettig oder verzweigtes (C1-C6)-Alkoxy, vorzugsweise Methoxy; geradkettig oder verzweigtes (C1-C6)-Alkyl, vorzugsweise Methyl; geradkettig oder verzweigtes (C1-C6)-Alkylendioxy (wobei das zweite Sauerstoffatom wahlweise der Rest R4 oder R6 sein kann), vorzugsweise Methylendioxy, Hydroxyl; mit einem oder mehreren Halogenatomen substituiertes geradkettig oder verzweigtes (C1-C6)-Alkoxy, vorzugsweise Trifluormethoxy; mit einem oder mehreren Halogenatomen substituiertes geradkettig oder verzweigtes (C1-C6)-Alkyl, vorzugsweise Trifluormethyl; steht.

4. Verwendung von mindestens einer Verbindung der allgemeinen Formel I nach einem der Ansprüche 1 bis 3, **dadurch gekennzeichnet, daß** R1, R2, R3, R5, R6, A, B, C, D, X und Y die vorstehend genannte Bedeutung haben und der Rest R4 für geradkettig oder verzweigtes (C1-C6)-Alkoxy, vorzugsweise Methoxy, steht.

5. Verwendung von mindestens einer Verbindung der allgemeinen Formel I nach einem der Ansprüche 1 bis 4, **dadurch gekennzeichnet, daß** R1, R2, R3, R5, R6, A, B, C, D, X und Y die vorstehend genannte Bedeutung haben und der Rest R4 für Methoxy steht.

6. Verwendung von mindestens einer Verbindung der allgemeinen Formel I nach einem der Ansprüche 1 bis 5, **dadurch gekennzeichnet, daß** R1-R6, A, B, C, D und X die vorstehend genannte Bedeutung haben und der Rest Y für substituiertes oder unsubstituiertes (C6-C14)-Aryl oder mindestens ein bis vier N, NH, O und/oder S als Ringglied aufweisendes (C1-C13)-Heteroaryl steht.

7. Verwendung von mindestens einer Verbindung der allgemeinen Formel I nach einem der Ansprüche 1 bis 6, **dadurch gekennzeichnet, daß** R1-R6, A, B, C, D und X die vorstehend genannte Bedeutung haben und der Rest Y für (C6-C14)-Aryl oder mindestens ein N, NH, O und/oder S als Ringglied aufweisendes (C1-C13)-Heteroaryl, welches unsubstituiert oder mit mindestens einem Rest ausgewählt aus der Gruppe bestehend aus Wasserstoff, Amino, Halogen, Nitro, Cyano, geradkettig oder verzweigtes (C1-C6)-Alkoxy, vorzugsweise Methoxy; geradkettig oder verzweigtes (C1-C6)-Alkyl, vorzugsweise Methyl; Hydroxy; (C1-C6)-Alkylcarbonyloxy, (C1-C6)-Alkoxycarbonyloxy; mit einem oder-mehreren Halogenatomen substituiertes geradkettig oder verzweigtes (C1-C6)-Alkoxy, vorzugsweise Trifluormethoxy; mit einem oder mehreren Halogenatomen substituiertes geradkettig oder verzweigtes (C1-C6)-Alkyl, vorzugsweise Trifluormethyl, substituiert ist, steht.

8. Verwendung von mindestens einer Verbindung der allgemeinen Formel I nach einem der Ansprüche 1 bis 7, **dadurch gekennzeichnet, daß** R1-R6, A, B, C, D und X die vorstehend genannte Bedeutung haben und der Rest Y für einen 1-Phenyl-Rest, welcher unsubstituiert oder mit Wasserstoff, 3,4-Dichlor, 2- oder 3-Methoxy, 2,4-Dimethoxy, 3-Nitro 3-Trifluormethyl, 2,3,4-Trimethoxy, 3,4,5-Trimethoxy substituiert ist, steht.

9. Verwendung nach einem der vorstehenden Ansprüche, **dadurch gekennzeichnet**, **das** das Arzneimittel zur Behandlung von Tumorerkrankungen eine antimitotische Wirkung in Säugetieren besitzt.

10. Verwendung nach einem der vorstehenden Ansprüche, **dadurch gekennzeichnet**, **das** das Arzneimittel zur Behandlung von Tumorerkrankungen eine direkte und/oder indirekte Inhibierung der Tubulinpolymerisation in Säuegetierzellen bewirkt.

11. Verwendung nach einem der vorstehenden Ansprüche, **dadurch gekennzeichnet, daß** das Arzneimittel zur Behandlung von Tumorerkrankungen zusätzlich übliche pharmazeutische Hilfs- und/oder Trägerstoffe enthält.

12. Verwendung nach einem der vorstehenden Ansprüche, **dadurch gekennzeichnet, daß** das Arzneimittel zur Behandlung von Tumorerkrankungen oral, parenteral oder topisch beim Säugetier, vorzugsweise beim Menschen, verabreicht werden kann.

## Claims

1. Use of at least one compound of the formula I 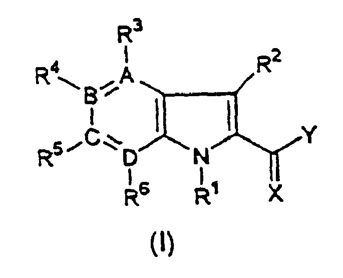 in which
R1 is hydrogen, (C1-C6)-alkylcarbonyl, preferably acetyl, (C₁-C₆)-alkyl, mono-(C₁-C₆)-alkylamino-(C₁-C₄)-alkyl, di-(C₁-C₆)-alkylamino-(C₁-C₄)-alkyl, where the two (C₁-C₆)-alkyl radicals together may form a ring, which optionally contains one or more NH, N-(C1-C6)-alkyl, 0 or S members, (C6-C14)-aryl-(C1-C6)-alkyl or (C6-C14)-aryl-(C1-C6)-alkoxy-(C1-C6)-alkyl;
R2 is a hydrogen atom, halogen, cyano, nitro, (C1-C6)-alkyl, (C1-C6)-alkyl which is substituted by one or more halogen atoms, preferably trifluoromethyl, (C1-C6)-alkoxy which is substituted by one or more halogen atoms, preferably trifluoromethoxy, (C2-C6)-alkenyl, (C2-C6)-alkynyl, (C3-C8)-cycloalkyl, (C1-C6)-alkoxy, (C1-C6)-alkoxycarbonyloxy, (C1-C6)-alkylcarbonyloxy, (C1-C4)-alkylthio, (C1-C4)-alkylsulfinyl, (C1-C4)-alkylsulfonyl, (C1-C6)-alkoxy-(C1-C6)-alkyl, amino, mono-(C1-C6)-alkylamino, di-N,N-(C1-C6)-alkylamino, where the two (C1-C6)-alkyl radicals together may form a ring, which optionally contains one or more NH, N-(C1-C6)-alkyl, O or S, (C6-C14)-aryl, (C6-C14)-aryloxy, (C6-C14)-aryl-(C1-C4)-alkyl, (C6-C14)-aryl-(C1-C4)-alkoxy-(C1-C4)-alkyl, (C1-C6)-alkylcarbonyl, (C1-C6)-alkoxycarbonyl or hydroxyl;
A, B, C and D independently of one another are a nitrogen atom (in which case R3, R4, R5 and R6 represent the free electron pair at the nitrogen atom) or are a carbon atom substituted by one of the radicals R3-R6 ;
R3, R4, R5 and R6 independently of one another are, when attached to nitrogen, a free electron pair, or, when attached to carbon, hydrogen, halogen, cyano, nitro, straight-chain or branched (C1-C6)-alkyl, straight-chain or branched (C1-C6)-alkyl which is substituted by one or more halogen atoms, preferably trifluoromethyl, straight-chain or branched (C1-C6)-alkoxy which is substituted by one or more halogen atoms, preferably trifluoromethoxy, (C2-C6)-alkenyl, (C2-C6)-alkynyl, (C3-C8)-cycloalkyl, straight-chain or branched (C1-C6)-alkoxy, preferably methoxy, straight-chain or branched (C1-C6)-alkylenedioxy, preferably methylenedioxy, (C1-C6)-alkoxycarbonyloxy, (C1-C6)-alkylcarbonyloxy, (C1-C4)-alkylthio, (C1-C4)-alkylsulfinyl, (C1-C4)-alkylsulfonyl, carboxyl, (C1-C6)-alkyl carboxylate, carboxamide, N-(C1-C4)-alkyl-carboxamide, N,N-di-(C1-C4)-alkylcarboxamide, (C1-C6)-alkoxy-(C1-C6)-alkyl, amino, mono-(C1-C6)-alkylamino, N,N-di-(C1-C6)-alkylamino, where the two C1-C6-alkyl radicals together may form a ring, which optionally contains one or more NH, N-(C1-C6)-alkyl, O or S, (C6-C14)-aryl, (C6-C14)-aryloxy, (C6-C14)-aryl-(C1-C4)-alkyl, (C6-C14)-aryl-(C1-C4)-alkoxy-(C1-C4)-alkyl, (C1-C6)-alkylcarbonyl, (C1-C6)-alkylcarbonyloxy, (C1-C6)-alkoxycarbonyl, hydroxyl, where two directly adjacent radicals may be attached to one another;
Y is unsubstituted (C6-C14)-aryl or (C6-C14)-aryl which is fully or partially substituted by identical or different substituents, preferably phenyl or 1- or 2-naphthyl, or is unsubstituted (C1-C13)-heteroaryl or (C1-C13)-heteroaryl which is fully or partially substituted by identical or different substituents and has in each case at least one to four N, NH, N-(C1-C6)-alkyl, 0 and/or S as ring members, or is unsubstituted (C3-C8)-cycloalkyl or (C3-C8)-cycloalkyl which is fully or partially substituted by identical or different substituents, where the identical or different substituents are selected independently of one another from the group consisting of halogen, preferably fluorine, chlorine, bromine or iodine; cyano; straight-chain or branched cyano-(C1-C6)-alkyl; hydroxyl; straight-chain or branched (C1-C6)-alkyl which is substituted by one or more hydroxyl groups; carboxyl; (C1-C6)-alkyl carboxylate, carboxamide; N-(C1-C6)-alkyl-carboxamide, N,N-di-(C1-C4)-alkyl-carboxamide, nitro, straight-chain or branched (C1-C6)-alkyl, straight-chain or branched (C1-C6)-alkyl which is substituted by one or more halogen atoms, preferably trifluoromethyl, straight-chain or branched (C1-C6)-alkoxy which is substituted by one or more halogen atoms, preferably trifluoromethoxy, straight-chain or branched (C2-C6)-alkenyl, straight-chain or branched (C2-C6)-alkynyl, (C3-C8)-cycloalkyl, straight-chain or branched (C1-C6)-alkoxy, preferably methoxy, straight-chain or branched (C1-C6)-alkylenedioxy, preferably methylenedioxy, thio (-SH), straight-chain or branched (C1-C6)-alkylthio, (C1-C6)-alkylsulfinyl, (C1-C5)-alkylsulfonyl, (C1-C6)-alkoxy-(C1-C6)-alkyl, amino, straight-chain or branched mono-(C1-C6)-alkylamino, straight-chain or branched N,N-di-(C1-C6)-alkylamino, where the two (C1-C6)-alkyl radicals together may form a ring, which may optionally contain one or more NH, N-(C1-C6)-alkyl, O and/or S, (C6-C14)-aryl, (C6-C14)-aryloxy, (C6-C14)-aryl-(C1-C6)-alkyl, (C6-C14)-aryl- (C1-C6) -alkoxy- (C1-C6) -alkyl, (C1-C6)-alkylcarbonyl, (C1-C6)-alkylcarbonyloxy, (C1-C6)-alkoxycarbonyl, (C1-C6)-alkoxycarbonyloxy, straight-chain or branched mono- and N,N-di-(C1-C6)-alkylcarbonylamino, straight-chain or branched mono- and N,N-di-(C1-C6)-alkoxycarbonylamino, straight-chain or branched N-(C1-C6)-alkylcarbonyl-N-(C1-C6)-alkylamino, straight-chain or branched N-(C1-C6)-alkoxycarbonyl-N-(C1-C6)-alkylamino, formylamino, formyl, where two directly adjacent radicals may be attached to one another;
X is an oxygen or sulfur atom, is NH, or is a geminally (at the same C atom) substituted hydroxyl and hydrogen (-CH(OH)-) ;
its stereoisomers, its tautomers, mixtures thereof and the pharmaceutically acceptable salts thereof, for preparing a medicament for the treatment of tumor diseases in mammals, preferably humans.

2. Use of at least one compound of the formula I according to claim 1, **characterized in that** R1-R6, A, B, C, D, X and Y are as defined in claim 1, with the proviso that at least one of the radicals R3-R6 is straight-chain or branched (C1-C6)-alkoxy, preferably methoxy; straight-chain or branched (C1-C6)-alkyl, preferably methyl; straight-chain or branched (C1-C6)-alkylenedioxy, preferably methylenedioxy, hydroxyl; straight-chain or branched (C1-C6)-alkoxy which is substituted by one or more halogen atoms, preferably trifluoromethoxy; straight-chain or branched (C1-C6)-alkyl which is substituted by one or more halogen atoms, preferably trifluoromethyl.

3. Use of at least one compound of the formula I according to claim 1, **characterized in that** that [sic] R1, R2, R3, R5, R6, A, B, C, D, X and Y are as defined above, with the proviso that the radical R4 is straight-chain or branched (C1-C6)-alkoxy, preferably methoxy; straight-chain or branched (C1-C6)-alkyl, preferably methyl; straight-chain or branched (C1-C6)-alkylenedioxy (where the second oxygen atom may optionally be the radical R4 or R6), preferably methylenedioxy, hydroxyl; straight-chain or branched (C1-C6)-alkoxy which is substituted by one or more halogen atoms, preferably trifluoromethoxy; straight-chain or branched (C1-C6)-alkyl which is substituted by one or more halogen atoms, preferably trifluoromethyl.

4. Use of at least one compound of the formula I according to claim 1, **characterized in that** that [sic] R1, R2, R3, R5, R6, A, B, C, D, X and Y are as defined above, with the proviso that the radical R4 is straight-chain or branched (C1-C6)-alkoxy, preferably methoxy.

5. Use of at least one compound of the formula I according to any of the preceding claims, **characterized in that** R1, R2, R3, R5, R6, A, B, C, D, X and Y are as defined above, with the proviso that the radical R4 is methoxy.

6. Use of at least one compound of the formula I according to any of the preceding claims, **characterized in that** R1-R6, A, B, C, D and X are as defined above, with the proviso that the radical Y is substituted or unsubstituted (C6-C14)-aryl or (C1-C13)-heteroaryl which contains at least one to four N, NH, 0 and/or S as ring members.

7. Use of at least one compound of the formula I according to any of the preceding claims, **characterized in that** R1-R6, A, B, C, D and X are as defined above, with the proviso that the radical Y is (C6-C14)-aryl or (C1-C13)-heteroaryl which contains at least one N, NH, 0 and/or S as ring members, which is substituted by at least one radical selected from the group consisting of hydrogen, amino, halogen, nitro, cyano, straight-chain or branched (C1-C6)-alkoxy, preferably methoxy; straight-chain or branched (C1-C6)-alkyl, preferably methyl; hydroxyl; (C1-C6)-alkylcarbonyloxy, (C1-C6)-alkoxycarbonyloxy; straight-chain or branched (C1-C6)-alkoxy which is substituted by one or more halogen atoms, preferably trifluoromethoxy; straight-chain or branched (C1-C6)-alkyl which is substituted by one or more halogen atoms, preferably trifluoromethyl.

8. Use of at least one compound of the formula I according to any of the preceding claims, **characterized in that** R1-R6, A, B, C, D and X are as defined above, **characterized in that** the radical Y is a 1-phenyl radical which is unsubstituted or substituted by hydrogen, 3,4-dichloro, 2- or 3-methoxy, 2,4-dimethoxy, 3-nitro[lacuna] 3-trifluoromethyl, 2,3,4-trimethoxy, 3,4,5-trimethoxy.

9. Use according to anyone of the preceding claims, **characterised in that** the medicament for the treatment of tumor diseases possesses an antimitotic action in mammals.

10. Use according to anyone of the preceding claims, **characterised in that** the medicament for the treatment of tumor diseases causes a direct and/or indirect inhibition of tubulin polymerization in mammalian cells.

11. Use according to anyone of the preceding claims, **characterised in that** the medicament for the treatment of tumor diseases additionally contains customary pharmaceutical auxiliaries and/or exhibitients.

12. Use according to anyone of the preceding claims, **characterised in that** the medicament for the treatment of tumor diseases can be administered orally, parenterally or topically to a mammal, preferably a human.

## Revendications

1. Utilisation d'au moins un composé de formule générale I : 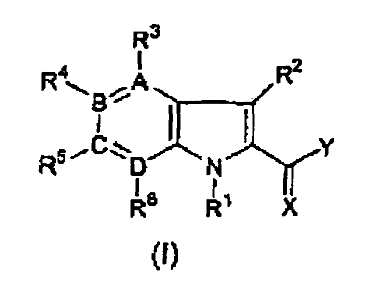 dans lequel :
R¹ représente un hydrogène, un (C₁-C₆)-alkylcarbonyle, de préférence un acétyle, un (C₁-C₆)-alkyle, un mono-(C₁-C₆)-alkylamino-(C₁-C₄)-alkyle, un di-(C₁-C₆)-alkylamino-(C₁-C₄)-alkyle, où les deux groupements (C₁-C₆)-alkyle peuvent former ensemble un cycle qui présente éventuellement un ou plusieurs chaînons NH, N-(C₁-C₆)-alkyle, O ou S, un (C₆-C₁₄)-aryl-(C₁-C₆)-alkyle ou un (C₆-C₁₄)-aryl-(C₁-C₆)-alkoxy-(C₁-C₆)-alkyle ;
R² représente un atome d'hydrogène, un halogène, un cyano, un nitro, un (C₁-C₆)-alkyle, un (C₁-C₆)-alkyle substitué par un ou plusieurs atomes d'halogène, de préférence un trifluorométhyle, un (C₁-C₆)-alkoxyle substitué par un ou plusieurs atomes d'halogène, de préférence un trifluorométhoxyle, un (C₂-C₆)-alcényle, un (C₂-C₆)-alcynyle, un (C₃-C₈)-cycloalkyle, un (C₁-C₆)-alkoxyle, un (C₁-C₆)-alkoxycarbonyloxyle, un (C₁-C₆)-alkylcarbonyloxyle, un (C₁-C₄)-alkylthiol, un (C₁-C₄)-alkylsulfinyle, un (C₁-C₄)-alkylsulfonyle, un (C₁-C₆)-alkoxy-(C₁-C₆)-alkyle, un amino, un mono-(C₁-C₆)-alkylamino, un N,N-(C₁-C₆)-dialkylamino, où les deux groupements (C₁-C₆)-alkyle peuvent former ensemble un cycle qui présente éventuellement un ou plusieurs chaînons NH, N-(C₁-C₆)-alkyle, O ou S, un (C₆-C₁₄)-aryle, un (C₆-C₁₄)-aryloxyle, un (C₆-C₁₄)-aryl-(C₁-C₄)-alkyle, un (C₆-C₁₄)-aryl-(C₁-C₄)-alkoxy-(C₁-C₄)-alkyle, un (C₁-C₆)-alkylcarbonyle, un (C₁-C₆)-alkoxycarbonyle ou un hydroxyle ;
A, B, C et D représentent indépendamment les uns des autres un atome d'azote (où R³, R⁴, R⁵ et R⁶ représentent alors le doublet électronique libre de l'atome d'azote) ou un atome de carbone substitué par l'un des groupements de R³ à R⁶ ;
R³, R⁴, R⁵ et R⁶ représentent indépendamment les uns des autres, un doublet électronique libre s'ils sont liés à un atome d'azote ou, s'ils sont liés à un atome de carbone, un hydrogène, un halogène, un cyano, un nitro, un (C₁-C₆)-alkyle linéaire ou ramifié, un (C₁-C₆)-alkyle linéaire ou ramifié substitué par un ou plusieurs atomes d'halogène, de préférence un trifluorométhyle, un (C₁-C₆)-alkoxyle linéaire ou ramifié substitué par un ou plusieurs atomes d'halogène, de préférence un trifluorométhoxyle, un (C₂-C₆)-alcényle, un (C₂-C₆)-alcynyle, un (C₃-C₈)-cycloalkyle, un (C₁-C₆)-alkoxyle linéaire ou ramifié, de préférence un méthoxyle, un (C₁-C₆)-alkylènedioxyle linéaire ou ramifié, de préférence un méthylènedioxyle, un (C₁-C₆)-alkoxycarbonyloxyle, un (C₁-C₆)-alkylcarbonyloxyle, un (C₁-C₄)-alkylthiol, un (C₁-C₄)-alkylsulfinyle, un (C₁-C₄)-alkylsulfonyle, un carboxyle, un carboxyester de (C₁-C₆)-alkyle, un carboxamide, un N-(C₁-C₄)-alkylcarboxamide, un N,N-(C₁-C₄)-dialkylcarboxamide, un (C₁-C₆)-alkoxy-(C₁-C₆)-alkyle, un mono-(C₁-C₆)-alkylamino, un N,N-(C₁-C₆)-dialkylamino, où les deux groupements (C₁-C₆)-alkyle peuvent former un ensemble cycle qui présente éventuellement un ou plusieurs chaînons NH, N-(C₁-C₆)-alkyle, O ou S, un (C₆-C₁₄)-aryle, un (C₆-C₁₄)-aryloxyle, un (C₆-C₁₄)-aryl-(C₁-C₄)-alkyle, un (C₆-C₁₄)-aryl-(C₁-C₄)-alkoxy-(C₁-C₄)-alkyle, un (C₁-C₆)-alkylcarbonyle, un (C₁-C₆)-alkylcarbonyloxyle, un (C₁-C₆)-alkoxycarbonyle, un hydroxyle, où deux groupements directement voisins peuvent être liés l'un à l'autre ;
Y représente un (C₆-C₁₄)-aryle non substitué ou partiellement ou totalement substitué par des substituants identiques ou différents, de préférence un phényle ou un 1- ou 2-naphtyle, ou un (C₁-C₁₃)-hétéroaryle non substitué ou partiellement ou totalement substitué par des substituants identiques ou différents, présentant au moins de un à quatre chaînons N, NH, N-(C₁-C₆)-alkyle, O et/ou S, ou un (C₃-C₈)-cycloalkyle non substitué ou partiellement ou totalement substitué par des substituants identiques ou différents, où les substituants identiques ou différents sont choisis indépendamment les uns des autres dans le groupe constitué d'un halogène, de préférence un fluor, un chlore, un brome ou un iode ; un cyano ; un (C₁-C₆)-cyanoalkyle linéaire ou ramifié ; un hydroxyle ; un (C₁-C₆)-alkyle linéaire ou ramifié substitué par un ou plusieurs hydroxyles ; un carboxyle ; un carboxyester de (C₁-C₆)-alkyle, un carboxamide ; un N-(C₁-C₆)-alkylcarboxamide ; un N,N-(C₁-C₆)-dialkylcarboxamide ; un nitro ; un (C₁-C₆)-alkyle linéaire ou ramifié, un (C₁-C₆)-alkyle linéaire ou ramifié substitué par un ou plusieurs atomes d'halogène, de préférence un trifluorométhyle ; un (C₁-C₆)-alkoxyle linéaire ou ramifié substitué par un ou plusieurs atomes d'halogène, de préférence un trifluorométhoxyle, un (C₂-C₆)-alcényle linéaire ou ramifié ; un (C₂-C₆)-alcynyle linéaire ou ramifié ; un (C₃-C₈)-cycloalkyle ; un (C₁-C₆)-alkoxyle linéaire ou ramifié, de préférence un méthoxyle, un (C₁-C₆)-alkylènedioxyle linéaire ou ramifié, de préférence un méthylènedioxyle, un thiol (-SH), un (C₁-C₆)-alkylthiol linéaire ou ramifié ; un (C₁-C₆)-alkylsulfinyle ; un (C₁-C₆)-alkylsulfonyle ; un (C₁-C₆)-alkoxy-(C₁-C₆)-alkyle ; un amino ; un mono-(C₁-C₆)-alkylamino linéaire ou ramifié ; un N,N-(C₁-C₆)-dialkylamino linéaire ou ramifié, où les deux groupements (C₁-C₆)-alkyle peuvent former ensemble un cycle qui présente éventuellement un ou plusieurs chaînons NH, N-(C₁-C₆)-alkyles, O et/ou S, un (C₆-C₁₄)-aryle, un (C₆-C₁₄)-aryloxyle, un (C₆-C₁₄)-aryle-(C₁-C₆)-alkyle, un (C₆-C₁₄)-aryl-(C₁-C₆)-alkoxy-(C₁-C₆)-alkyle, un (C₁-C₆)-alkylcarbonyle, un (C₁-C₆)-alkylcarbonyloxyle, un (C₁-C₆)-alkoxycarbonyle, un (C₁-C₆)-alkoxycarbonyloxyle, un mono- ou N,N-di-(C₁-C₆)-alkylcarbonylamino linéaire ou ramifié, un mono- ou N,N-di-(C₁-C₆)-alkoxycarbonylamino linéaire ou ramifié, un N-(C₁-C₆)-alkylcarbonyl-N-(C₁-C₆)-alkylamino linéaire ou ramifié, un N-(C₁-C₆)-alkoxy-carbonyl-N-(C₁-C₆)-alkylamino linéaire ou ramifié, un formylamino, un formyle, où deux groupements directement voisins peuvent être liés l'un à l'autre ;
X représente un atome d'oxygène ou de soufre, NH, ou un atome de carbone substitué géminalement (sur le même atome de carbone) par un hydroxyle et un hydrogène (-CH(OH)-) ;
leurs stéréoisomères, leurs tautomères, leurs mélanges ainsi que leurs sels pharmaceutiquement compatibles pour la fabrication d'un médicament pour le traitement de maladies tumorales chez des mammifères, de préférence chez l'homme.

2. Utilisation d'au moins un composé de formule générale I selon la revendication 1 **caractérisée en ce que** R¹ à R⁶, A, B, C, D, X et Y ont la signification indiquée à la revendication 1 dans la mesure où au moins un des groupements R³ à R⁶ représente un (C₁-C₆)-alkoxyle linéaire ou ramifié, de préférence un méthoxyle, un (C₁-C₆)-alkyle linéaire ou ramifié, de préférence un méthyle ; un (C₁-C₆)-alkylènedioxyle linéaire ou ramifié, de préférence un méthylènedioxyle ; un hydroxyle, un (C₁-C₆)-alkoxyle linéaire ou ramifié substitué par un ou plusieurs atomes d'halogène, de préférence un trifluorométhoxyle ; ou un (C₁-C₆)-alkyle linéaire ou ramifié substitué par un ou plusieurs atomes d'halogène, de préférence un trifluorométhyle.

3. Utilisation d'au moins un composé de formule générale I selon la revendication 1 ou 2, **caractérisée en ce que** R¹, R², R³, R⁵, R⁶, A, B, C, D, X et Y ont la signification indiquée ci-dessus et le groupement R⁴ représente un (C₁-C₆)-alkoxyle linéaire ou ramifié, de préférence un méthoxyle ; un (C₁-C₆)-alkyle linéaire ou ramifié, de préférence un méthyle ; un (C₁-C₆)-alkylènedioxyle linéaire ou ramifié (où le deuxième atome d'oxygène peut être au choix le groupement R⁴ ou R⁶), de préférence un méthylènedioxyle, un hydroxyle ; un (C₁-C₆)-alkoxyle linéaire ou ramifié substitué par un ou plusieurs atomes d'halogène, de préférence un trifluorométhoxyle ; ou un (C₁-C₆)-alkyle linéaire ou ramifié substitué par un ou plusieurs atomes d'halogène, de préférence un trifluorométhyle.

4. Utilisation d'au moins un composé de formule générale I selon l'une quelconque des revendications 1 à 3, **caractérisée en ce que** R¹, R², R³, R⁵, R⁶, A, B, C, D, X et Y ont la signification indiquée ci-dessus et R⁴ représente un (C₁-C₆)-alkoxyle linéaire ou ramifié, de préférence un méthoxyle.

5. Utilisation d'au moins un composé de formule générale I selon l'une quelconque des revendications 1 à 4, **caractérisée en ce que** R¹, R², R³, R⁵, R⁶, A, B, C, D, X et Y ont la signification indiquée ci-dessus et R⁴ représente un méthoxyle.

6. Utilisation d'au moins un composé de formule générale I selon l'une quelconque des revendications 1 à 5, **caractérisée en ce que** R¹ à R⁶, A, B, C, D et X ont la signification indiquée ci-dessus et Y représente un (C₆-C₁₄)-aryle substitué ou non substitué, ou un (C₁-C₁₃)-hétéroaryle présentant au moins de un à quatre chaînons N, NH, O et/ou S.

7. Utilisation d'au moins un composé de formule générale 1 selon l'une quelconque des revendications 1 à 6, **caractérisée en ce que** R¹ à R⁶, A, B, C, D et X ont la signification indiquée ci-dessus et Y représente un (C₆-C₁₄)-aryle ou un (C₁-C₁₃)-hétéroaryle présentant au moins un chaînon N, NH, O et/ou S, qui est non substitué ou avec au moins un groupement choisi dans le groupe constitué d'un hydrogène, un amino, un halogène, un nitro, un cyano, un (C₁-C₆)-alkoxyle linéaire ou ramifié, de préférence un méthoxyle, un (C₁-C₆)-alkyle linéaire ou ramifié, de préférence un méthyle, un hydroxyle, un (C₁-C₆)-alkylcarbonyloxyle, un (C₁-C₆)-alkoxycarbonyloxyle, un (C₁-C₆)-alkoxyle linéaire ou ramifié substitué par un ou plusieurs atomes d'halogène, de préférence un trifluorométhoxyle, ou un (C₁-C₆)-alkyle substitué par un ou plusieurs atomes d'halogène, de préférence un trifluorométhyle.

8. Utilisation d'au moins un composé de formule générale I selon l'une quelconque des revendications 1 à 7, **caractérisée en ce que** R¹ à R⁶, A, B, C, D et X ont la signification indiquée ci-dessus et Y représente un groupement 1-phényle, non substitué ou substitué par un hydrogène, un 3,4-dichloro, un 2- ou 3-méthoxyle, un 2,4-diméthoxyle, un 3-nitro, un 3-trifluorométhyle, un 2,3,4-triméthoxyle ou un 3,4,5-triméthoxyle.

9. Utilisation selon l'une quelconque des revendications précédentes, **caractérisée en ce que** le médicament possède une action antimitotique pour le traitement de maladies tumorales chez des mammifères.

10. Utilisation selon l'une quelconque des revendications précédentes, **caractérisée en ce que** le médicament entraîne une inhibition directe et/ou indirecte de la polymérisation de la tubuline pour le traitement de maladies tumorales dans des cellules de mammifères.

11. Utilisation selon l'une quelconque des revendications précédentes, **caractérisée en ce que** le médicament contient en plus des adjuvants et/ou des substances vectrices pharmaceutiques habituels pour le traitement de maladies tumorales.

12. Utilisation selon l'une quelconque des revendications précédentes, **caractérisée en ce que** le médicament peut être administré par voie orale, parentérale ou topique chez le mammifère, de préférence chez l'homme, pour le traitement de maladies tumorales.
